# EUROPEAN PATENT APPLICATION

(11) **EP 4 751 656 A1**
(43) Date of publication of application: **03.06.2026**
(21) Application number: 24215710.5
(22) Date of filing: 27.11.2024
(51) Int. Cl.: A61B 8/00

(54) **A SYSTEM AND A METHOD FOR LOCALIZATION OF AN ANATOMICAL PART IN A SUBJECT**

(71) Applicant: Imec VZW, 3001 Leuven (BE); Stichting IMEC Nederland, 5656 AE Eindhoven (NL)
(72) Inventor: BEUTEL, Fabian, 40547 Duesseldorf (DE); HUYGHE, Mario, 8792 Desselgem (BE)
(74) Representative: AWA Sweden AB

(57) **Abstract**

A system (100) for localization of an anatomical part in a subject comprises: an array of ultrasound transducers (110); a control unit (120) configured to activate a selected subset of at least one ultrasound transducer (110) for controlling the subset to assume a localization mode, wherein the at least one ultrasound transducer (110) is configured to transmit pulse(s) of ultrasound signals and detect the reflected pulse(s) for forming pulse echo data representing an ultrasound response for each pulse as a function of a time delay, wherein the control unit (120) is further configured to sequentially activate different selected subsets; a processing unit (130) configured to analyze the pulse echo data in relation to a characteristic pattern, and to identify one or more ultrasound transducers (110) being located to acquire pulse echo data representing the anatomical part for determining a location of the anatomical part in the subject.

## Description

### Technical field

The present description relates to localization of an anatomical part in a subject, such as localization of a blood vessel. In particular, the present description relates to a system and a method for localization of an anatomical part in a subject.

### Background

Cardiovascular disease is a common problem. It is therefore of large interest to enable estimating cardiovascular health of subjects in a simple and robust manner. In particular, if estimation of cardiovascular health can be provided early, such as before any symptoms are noticeable, health issues may be identified at an early stage.

Thus, it would be interesting to enable estimation of cardiovascular health to a large proportion of a population and to enable screening of cardiovascular health at an early stage. This implies that estimation of cardiovascular health should be performed in a simple, yet precise, manner. For instance, it would be desired to enable estimation of cardiovascular health without requiring involvement of a skilled medical practitioner, such as a physician or a nurse.

Ultrasound sensing may be used for providing visualization of structural information and functional physiology without a subject being exposed to ionizing radiation. However, ultrasound sensing typically requires a skilled medical practitioner to manually locate and direct an ultrasound transducer to a relevant anatomical region of interest. Subsequently, clinically relevant parameters, such as structural, anatomical, physiological, or functional parameters, may be extracted based on a semi-automated process, wherein the medical professional manually marks anatomical landmarks in order to allow automated extraction of information from the ultrasound sensing and computation of cardiovascular health information.

Thus, there is a need for an improvement in equipment for providing cardiovascular health information.

Although problems related to cardiovascular health assessment are discussed above, it should be realized that it would be of interest to provide an improved acquisition of information and/or estimation of a health parameter in relation to other anatomical parts.

### Summary

An objective of the present description is to enable simple, practical, and precise estimation of information relating to an anatomical part of a subject. A particular objective of the present description is to enable simple, practical, and precise estimation of cardiovascular health.

These and other objectives of the present inventive concept are at least partly met by the invention as defined in the independent claims. Preferred embodiments are set out in the dependent claims.

According to a first aspect, there is provided a system for localization of an anatomical part in a subject, said system comprising: an array of ultrasound transducers configured to transmit ultrasound signals into the subject and to detect reflected ultrasound signals from the subject; a control unit configured to activate a selected subset of at least one ultrasound transducer from the array of ultrasound transducers for controlling the subset to assume a localization mode, wherein the at least one ultrasound transducer in the selected subset is configured to transmit one or more pulses of ultrasound signals into the subject and to detect the reflected one or more pulses for forming pulse echo data representing an ultrasound response from the subject for each pulse as a function of a time delay of the ultrasound response, wherein the control unit is further configured to sequentially activate different selected subsets from the array of ultrasound transducers; a processing unit configured to analyze the pulse echo data acquired by a plurality of selected subsets in relation to a characteristic pattern of pulse echo representing a structure of the anatomical part, and to identify one or more ultrasound transducers being located to acquire pulse echo data representing the anatomical part for determining a location of the anatomical part in the subject.

The system enables localization of the anatomical part of the subject in a simple manner. The ultrasound transducers are configured to acquire pulse echo data from the subject. The pulse echo data may be directly received from the ultrasound transducers and there may be no need of complicated processing of signals detected by the ultrasound transducers for providing a representation of the anatomy of the subject in order to allow identification of the anatomical part. In particular, there is no need of constructing an image of the anatomy of the subject in order to allow the anatomical part to be identified in the image.

It should be realized that constructing an image from ultrasound information may require extensive processing resources such that a powerful processor may be needed, and large power consumption may be allowed.

Thanks to the system of the first aspect not requiring construction of an image for allowing localizing the anatomical part, the system may have a relatively large array of ultrasound transducers. Since data acquired by the array of ultrasound transducers may not need to be processed in a computationally extensive manner, a large array of ultrasound transducers being arranged to acquire information may be allowed.

Thanks to using an array of ultrasound transducers, the system provides lenient requirements on placement of the array of ultrasound transducers in relation to the anatomical part of interest. Thus, even if the array of ultrasound transducers is not perfectly aligned with the anatomical part, at least some part of the array may be able to detect information from the anatomical part so as to allow localization of the anatomical part.

According to an embodiment, based on the processing unit identifying that the characteristic pattern of pulse echo is present in the pulse echo data, the control unit is further configured to terminate sequential activation of selected subsets from the array of ultrasound transducers.

Thus, it should be realized that only a part of the array may need to be activated for finding the anatomical part of interest. Once found, further activation for iterating through the entire array of ultrasound transducers is not needed.

It should further be realized that, when the characteristic pattern representing the structure of the anatomical part is identified, this may be used for controlling further activation of selected subsets in order to allow identifying ultrasound transducers in the array that are arranged along an extension of the anatomical part. Thereafter, sequential activation of the selected subsets may be terminated.

The system for localization of the anatomical part may provide a location of the anatomical part in relation to an arrangement of the array of ultrasound transducers. Thus, the array of ultrasound transducers may be configured to be arranged externally to the subject, such as in contact with skin. However, it should be realized that the array of ultrasound transducers may alternatively be configured to at least partially implanted in a subject. In such case, the control unit and/or the processing unit may in some embodiments also be arranged in a housing that is implanted in the subject but may in other embodiments be arranged externally to the subject.

The system may further be configured to determine the location of the anatomical part in relation to a position of the array of ultrasound transducers. Hence, it should be realized that the localization of the anatomical part need not necessarily provide an absolute localization of the anatomical part but rather a relative location of the anatomical part in relation to the array of ultrasound transducers.

It should further be realized that the localization of the anatomical part may be useful for enabling further analysis of the anatomical part. Thus, once the anatomical part is localized, information for allowing estimation of a health condition may be properly acquired. In addition, the array of ultrasound transducers may also be used for acquiring information allowing assessment of a health condition, after the anatomical part has been localized. Therefore, by enabling localization of the anatomical part in a simple and robust manner that may not need any action by a skilled medical practitioner, the array of ultrasound transducers may be properly placed in relation to the anatomical part of interest. This may further be used for ensuring that information for assessing the health condition may be acquired in a reliable manner as the relation between the array of ultrasound transducers and the anatomical part is known. Hence, the estimation of the health condition can also be reliably performed, possibly using automated acquisition of information and estimation of the health condition. Therefore, the system may enable automated assessment of a health condition without requiring involvement of a skilled medical practitioner for placement of a sensor or for analysis of acquired information.

It should further be realized that the system may comprise additional sensor(s), which may be arranged in a known relation to the array of ultrasound transducers. Thus, information for allowing assessing the health condition may be acquired by such additional sensors and not necessarily by the array of ultrasound transducers. As yet another alternative, the array of ultrasound transducers may be used in combination with the additional sensors for acquiring the information for allowing assessing the health condition.

The anatomical part may be a part of a vascular system, such as a blood vessel. For instance, the anatomical part may be an artery. The system may thus be configured to localize a part of the vascular system allowing further parameters relating to cardiovascular health assessment to be acquired using the localization information. However, it should be realized that the anatomical part could be another part of a body, such as any organ in the subject. The subject may be a human being or an animal.

The ultrasound transducers may be arranged in a regular manner in the array. Thus, a distance between adjacent ultrasound transducers may be equal throughout the array. The ultrasound transducers may be arranged in a one-dimensional array or a two-dimensional array. In case of a two-dimensional array, the distance between adjacent ultrasound transducers may be equal along rows and columns. Alternatively, the ultrasound transducers may be more densely arranged in one direction than the other direction. It should further be realized that the ultrasound transducers may alternatively be arranged in an irregular array.

The array of ultrasound transducers may be carried by a common carrier, such that all of the ultrasound transducers may be arranged in relation to the subject in a single act of placing the array of ultrasound transducers. However, according to an alternative, the array of ultrasound transducers may comprise a plurality of subsets, each comprising at least one ultrasound transducer, wherein each subset may be separately positioned in relation to the subject.

It should further be realized that a carrier may be flexible so as to allow the carrier to conform to a shape of a body part of the subject. The array of ultrasound transducers may thus be arranged on a flexible substrate. Hence, a relation between individual ultrasound transducers may be changed when the carrier is arranged in relation to the subject.

It should further be realized that the ultrasound transducers in the array may at least in some embodiments, during localization, be considered to have a fixed relation to each other and to the anatomical part in order to allow the anatomical part to be localized in relation to the array of ultrasound transducers. It should however be realized that the fixed relation could still imply that the ultrasound transducers are subject to motion artefacts (e.g., due to breathing) that may affect not only the relation between the ultrasound transducers but also the relation between the ultrasound transducers and the anatomical part. Also, it should be realized that during activation of a selected subset, the ultrasound transducers may be considered to have a fixed relation. However, if the entire array or a large part of the array is scanned, the ultrasound transducers may not necessarily have a fixed relation during such scanning. Nevertheless, a localization of the anatomical part in relation to the array of ultrasound transducers is enabled.

Thus, according to an embodiment, the array of ultrasound transducers is configured to be carried by a carrier for placing the array of ultrasound transducers in relation to the anatomical part, wherein the carrier is flexible to allow the carrier to conform to a shape of the body part. Thus, the carrier may be able to bend for changing shape and may also possibly be able to be stretched for conforming to the shape of the body part. Each ultrasound transducer may have a rigid shape, wherein a flexible carrier is arranged between the ultrasound transducers for allowing the array of ultrasound transducers to be arranged in a flexible relation to allow the array of ultrasound transducers to conform to the shape of the body part.

The carrier may further comprise a flex sensor configured to sense a change in shape of the carrier. Thus, the flex sensor may be configured to sense any change in shape from a planar shape. The flex sensor may for instance comprise a resistive, capacitive, or optical sensor for sensing changes in shape of the carrier. Information from the flex sensor may allow determination of the relation between different ultrasound transducers in the array, which may be used for determining relative information of pulse echo data from different ultrasound transducers and for properly controlling beamforming by a plurality of ultrasound transducers.

It should further be realized that the array of ultrasound sensors may be arranged in a rigid planar arrangement. The system may further comprise an interface layer which may be configured to be arranged between the array of ultrasound transducers and the body part, wherein the interface layer may be compressible for allowing the interface layer to conform to the shape of the body part.

The ultrasound transducers may be any type of transducer configured to convert a signal, such as an electrical or optical signal, to ultrasound and also to convert a received ultrasound signal back to a signal that may be recorded, such as an electrical or optical signal. An ultrasound signal transmitted by the ultrasound transducer may be controlled by controlling the signal, such as the electrical signal, provided to the ultrasound transducer. The signal generated by the ultrasound transducer may further be recorded by a detector for generating data based on received ultrasound signals. The ultrasound transducer may comprise a membrane for transmitting ultrasound and also being sensitive to received ultrasound.

The ultrasound transducer may for instance be formed as a piezoelectric or capacitive transducer. Each ultrasound transducer may further be small and may be formed as a piezo-electric micro-machined ultrasound transducer (PMUT), a capacitive micro-machined ultrasound transducer (CMUT), or an optical micro-machined ultrasound sensor (OMUS). The size of each ultrasonic transducer may for instance be in an order of 100 µm, or even smaller, such as smaller than 50 µm.

The ultrasound transducers may be configured to make contact with the subject for transmitting ultrasound signals into tissue and for receiving reflected ultrasound signals propagating in the tissue. The ultrasound transducers may be provided with an interface medium to be arranged between the ultrasound transducers and the tissue for facilitating efficient transfer of ultrasound into and out of tissue. The interface medium may for instance be provided permanently attached to the array of ultrasound transducers but may alternatively be applied when the array of ultrasound transducers is to be used.

According to an embodiment, the array of ultrasound transducers may further be provided with a protective layer arranged on the array of ultrasound transducers, such that the protective layer is configured to be arranged between the array of ultrasound transducers and the body part, when the array of ultrasound transducers is used.

The array of ultrasound transducers may further be configured to receive an interface element comprising the interface medium. If the array of ultrasound transducers comprises a protective layer, the interface medium may be configured to be arranged on the protective layer.

For instance, the array of ultrasound transducers may be configured to be carried by a carrier, wherein the carrier may comprise a guiding structure for guiding placement of the interface element on the carrier. The carrier could, e.g., have a frame structure around the array of ultrasound transducers such that a recess is defined on the array of ultrasound transducers. The interface element may thus be arranged in the recess so as to properly place the interface medium between the array of ultrasound transducers and the body part when the array of ultrasound transducers is used.

The interface element could for instance be a disposable hydrogel pad. Thus, the interface element may be replaced each time the array of ultrasound transducers is to be used.

The control unit may be configured to generate control signals that may be output to the selected subset of ultrasound transducer(s). The control signals may thus cause transmission of the ultrasound transducer(s) receiving the control signals.

The control unit may be configured to control a sequence in which subsets of ultrasound transducer(s) are activated. Sequential activation may allow all of the ultrasound transducers in the array to be sequentially activated in an orderly fashion for scanning the entire array of ultrasound transducers.

Thus, according to an embodiment, the control unit is configured to sequentially activate different selected subsets for scanning the array of ultrasound transducers. Each ultrasound transducer will be activated during scanning of the array such that the entire array of ultrasound transducers is scanned.

However, the control unit may also or alternatively select subsets to be activated in dependence of detected pulse echo data. In such case, not all ultrasound transducers may need to be activated, for instance if the anatomical part is localized in relation to a particular subset of ultrasound transducers.

The control unit may in some embodiments be configured to activate a row or a column of ultrasound transducers in the array at a time providing row-wise or column-wise scanning. It should also be realized that other subsets may be activated, such as subsets extending diagonally in the array, subsets including only part of a row, column, or diagonal, or two-dimensional subsets. In addition, each subset may even correspond only to a single ultrasound transducer.

It should further be realized that the ultrasound transducers in the selected subset may be controlled in various manners for transmitting and receiving ultrasound. For instance, all of the ultrasound transducers in the selected subset may both transmit ultrasound pulse(s) and detect pulse echo. However, in other embodiments, not all of the ultrasound transducers are used for both transmitting pulse(s) and detecting pulse echo. Rather, many (e.g., all) of the ultrasound transducers in the subset may be used for transmitting ultrasound pulse(s), whereas only a few (e.g., one) ultrasound transducers are used for detecting the pulse echo, or only a few (e.g., one) ultrasound transducers are used for transmitting ultrasound pulse(s), whereas many (e.g., all) ultrasound transducers in the subset are used for detecting the pulse echo.

The selected ultrasound transducers in a subset are configured to transmit ultrasound pulse(s) and detect pulse echo data. The control unit may further be configured to control a relation between the ultrasound transducers in the subset for transmitting ultrasound pulse(s) and detecting pulse echo data in order to control steering a direction of the ultrasound in tissue in relation to the array of ultrasound transducers. This control may be used for controlling a direction of a plane wave of ultrasound into tissue or for controlling a focus of the ultrasound in the tissue.

Thus, it should be realized that an amplitude of a received pulse echo (i.e., reflected ultrasound) may be detected as a function of time. The pulse echo data may thus represent the ultrasound response from the subject as a function of time delay from a time point at which the pulse was output. The amplitude of the received pulse echo at a particular time delay may represent an amount of reflection of ultrasound at a particular depth into tissue of the subject, wherein a travel time of the ultrasound pulse to the particular depth and back to the ultrasound transducer corresponds to the particular time delay.

The ultrasound transducers may be configured to transmit a single pulse of ultrasound signal. It should be realized that a single pulse is sufficient for forming pulse echo data. However, the ultrasound transducers may transmit more than one pulse. This may be used for providing more accurate information such as for improving a signal-to-noise ratio by combining pulse echoes from several pulses. Also, transmitting more than one pulse may allow following changes of the location of the anatomical part.

It should be realized that the pulse echo data provides a representation of the received ultrasound echo as a function of time. The pulse echo data received by different ultrasound transducers in the subset may possibly be processed for comparing the relation of pulse echo in different ultrasound transducers, allowing information of an origin of the pulse echo in relation to the array of ultrasound transducers to be deduced. For a rigid array of ultrasound transducers, the origin of the pulse echo may thus provide information of a location of the anatomical part in relation to the array. For a flexible arrangement of the ultrasound transducers in relation to each other, information of the relation between the ultrasound transducers (e.g., from a flex sensor) may be used in combination with information of the origin of the pulse echo for determining a location of the anatomical part in relation to the array.

However, the pulse echo data is merely a representation of intensity of pulse echo as a function of time, as recorded in one or more ultrasound transducers of the subset. The pulse echo data may thus be represented as a time-varying signal. It should be further realized that the recording of pulse echo data may be referred to as A-mode (amplitude-mode) ultrasound detection.

The processing unit is configured to analyze the pulse echo data. The pulse echo data is acquired by the ultrasound transducer(s) in the selected subset by the transmission of pulse(s) and detection of the pulse echo. The processing unit is configured to analyze the pulse echo data in relation to a characteristic pattern. This implies that the processing unit is configured to analyze the pulse echo data without requiring the pulse echo data first to be processed in order to generate an image of the tissue of the subject.

The processing unit may analyze the pulse echo data using a characteristic pattern of pulse echo representing the anatomical part. Thus, a characteristic pattern of the anatomical part of interest may be known to the processing unit for allowing the processing unit to determine whether the characteristic pattern is present in the pulse echo data. The characteristic pattern may be stored in a memory accessible to the processing unit allowing the processing unit to directly compare the pulse echo data to the characteristic pattern. Alternatively, the characteristic pattern may be used for training a neural network such that the processing unit may use the neural network trained using the characteristic pattern for processing the pulse echo data.

The characteristic pattern of the pulse echo may represent characteristics of a structure of the anatomical part. Thus, the anatomical part may provide a characteristic response in the pulse echo based on its structure.

For instance, if the anatomical part to be located is a part of the vascular structure, such as a blood vessel, the characteristic pattern may correspond to two amplitude peaks separated by a hypoechoic portion. The blood vessel may provide strong echo from walls of the blood vessel but almost no reflection from the interior of the blood vessel. Thus, the characteristic pattern forms a representation of a variation in pulse echo in the detected time signal, which may be detected in the pulse echo data without requiring constructing an image. It should be realized that corresponding characteristic patterns may be determined for other anatomical parts to be localized.

The processing unit may be configured to determine ultrasound transducers among the array of ultrasound transducers that detect pulse echo data comprising the characteristic pattern. Thus, the location of the anatomical part may be determined by determining which ultrasound transducers that detect the pulse echo data that comprises the characteristic pattern.

The array of ultrasound transducers may define lateral directions extending along a displacement of ultrasound transducers in relation to each other in the array. For instance, if the array of ultrasound transducers is arranged in a plane, the lateral directions also extend in the plane. However, it should be realized that the array may not necessarily be arranged in a plane but may for instance conform to a curved shape of a body part. The location of the anatomical part may be determined in the lateral directions, such as in relation to a plane parallel to the array of ultrasound transducers. This determination may be based on the identification of which ultrasound transducers that detect the pulse echo data.

It should further be realized that the determining of the location of the anatomical part may provide a localization of an extension of the anatomical part. Thus, at least in some embodiments, the system may be configured to determine at least two different points on the anatomical part in relation to the lateral directions. For instance, if the anatomical part is a blood vessel, the system may be configured to determine an extension of the blood vessel along the lateral directions, or at least determine locations of two different longitudinal positions in the blood vessel. However, it should be realized that in other embodiments a single location of the anatomical part may be determined.

The location of the anatomical part based on identifying ultrasound transducers that detect the pulse echo data may further be complemented by determining a depth location, i.e., a location in relation to a direction perpendicular to the lateral directions, such as a direction perpendicular to the plane of the array. The depth location may be determined based on a time delay in the pulse echo data corresponding to the characteristic pattern.

The ultrasound transducers may be set to the localization mode for allowing localization of the anatomical part. In the localization mode, the ultrasound transducers are configured to transmit ultrasound pulses and detect pulse echo data. It should further be realized that the ultrasound transducers may be set to other modes, which may be used for acquiring complementary information to the localization of the anatomical part. For instance, once the anatomical part has been localized, some of the ultrasound transducers may be used in other modes, such as a Doppler mode, for determining further information of the anatomical part. It should however also be realized that the ultrasound transducers may only be used for locating the anatomical part and may in such case only be used in the localization mode.

It should be realized that the control unit may be implemented in a general-purpose processing unit, such as a central processing unit (CPU), which may execute instructions of one or more computer programs in order to implement functionality of the control unit. The control unit may alternatively be implemented as firmware arranged e.g., in an embedded system, or as a specifically designed processing unit, such as an Application-Specific Integrated Circuit (ASIC) or a Field-Programmable Gate Array (FPGA).

Similarly, the processing unit may be implemented in a general-purpose processing unit, such as a CPU, which may execute instructions of one or more computer programs in order to implement functionality of the processing unit. The processing unit may alternatively be implemented as firmware arranged e.g., in an embedded system, or as a specifically designed processing unit, such as an ASIC or an FPGA.

The processing unit and the control unit may be commonly implemented in the general-purpose processing unit. Thus, the processing unit and the control unit may be implemented by different software modules or different execution threads executed by the general-purpose processing unit.

It should further be realized that the control unit may comprise one or more digital-to-analog converters for converting control signals to analog domain for providing the control signals to the ultrasound transducers. However, it should further be realized that digital-to-analog converters may alternatively be implemented in the ultrasound transducers.

The control unit may be arranged close to the array of ultrasound transducers in order to allow fast transport of control signals to the ultrasound transducers. The control unit may be connected by wires to the array of ultrasound transducers.

The processing unit may also be arranged close to the control unit and close to the array of ultrasound transducers and the processing unit may be connected to the control unit by wires or by being implemented as different modules in a common general-purpose processing unit.

Thus, both the control unit and the processing unit may be arranged in a common housing with the array of ultrasound transducers. However, it should be realized that the control unit and the processing unit may be arranged in different locations in relation to the array of ultrasound transducers. For instance, the control unit and/or the processing unit may be distributed onto several physical units. Both the control unit and the processing unit, but the processing unit in particular, may be arranged in a separate housing from the array of ultrasound transducers and may be configured to provide communication through wired or wireless communication. For instance, the processing unit may be arranged in a separate housing from the control unit and may be configured to communicate with the control unit through wired or wireless communication. The processing unit may, e.g., be arranged in vicinity to the control unit such as in a wearable or portable unit that the subject may be wearing, such as in a mobile phone or a smart watch. However, the processing unit need not even be arranged in vicinity to the control unit or the array of ultrasound transducers but may rather be arranged anywhere, such as in a server "in the cloud".

It should be realized that even though the system is described above to not require construction of an image of the tissue for enabling determining the location of the anatomical part, this does not exclude that the system is still used for generating an image. For instance, an image may be generated for providing a visualization to a user even though the image need not be used by the processing unit for determining the location of the anatomical part. In addition, the array of ultrasound transducers may be used for acquiring further information for generating an image once the anatomical part has been located. Thus, after the location of the anatomical part, some (or all) of the ultrasound transducer may be set to a different mode in which ultrasound information is acquired for generating an image.

According to an embodiment, the system further comprises: a carrier configured to be adjustably positioned in relation to a body part, such as around the body part, wherein the array of ultrasound transducers is configured to be carried by the carrier for placing the array of ultrasound transducers in relation to the anatomical part.

The carrier may be configured to enable simple re-positioning of the carrier in relation to the body part. This implies that the carrier need not be properly or perfectly placed in a first attempt to arrange the array of ultrasound transducers in relation to the anatomical part.

The system may be configured to use a relatively large array of ultrasound transducers in combination with an adjustable carrier. This may allow the array of ultrasound transducers to be positioned such that the anatomical part may be reliably located, at least in a few attempts of placing the array of ultrasound transducer in relation to the body part. This may also ensure that the system may be used by a person that is not a skilled medical practitioner. For instance, the system may be used by the subject placing the array of ultrasound transducers in relation to the body part. This facilitates using the system for reliable self-assessment of health. This may substantially improve screening for common diseases, such as diseases related to the cardiovascular system.

The body part may be any suitable body part that is close to the anatomical part to be located. For instance, the body part may be a neck, a torso, an arm, or a leg. The carrier may be arranged around the body part. This may be useful for providing a steady positioning of the carrier and also for providing a simple arrangement of the carrier in relation to the body part. This may further be useful since no adhesive may be needed for maintaining the position of the carrier in relation to the body part and since no adhesive need be used, numerous attempts of adjustably positioning the carrier may be allowed.

Even though the carrier may be arranged around a body part, the carrier may not need to be arranged completely enclosing the body part. This may still allow steady positioning of the carrier and simple adjustment of the position of the array of ultrasound transducers in relation to the body part.

It should further be realized that the carrier need not necessarily be arranged around the body part. Rather, the carrier may be configured to be adjustably positioned on a portion of a surface of the skin, such as on a torso. The carrier may be adjustably positioned using an adhesive surface that may be re-used for providing adhesion to skin in multiple attempts. The carrier may further be positioned in relation to the body part in another manner. For instance, one or more suction cups may be used for forming a local low pressure to attach the carrier to the skin.

According to an embodiment, the carrier is configured to be strapped around the body part.

This may provide a very simple manner of positioning the array of ultrasound transducer in relation to the anatomical part. The carrier may thus be easily tightened around the body part to fit different sizes of subjects and to ensure a proper contact between the ultrasound transducers and skin of the subject. In addition, the carrier may be easily released for providing re-positioning of the array of ultrasound transducers in relation to the anatomical part.

For instance, the carrier may be provided in form of a belt, a cuff, or a Posey strap.

According to another embodiment, the carrier comprises a structure configured to be arranged extending around the body part, wherein the structure has an opening for not fully enclosing the body part.

The structure may be configured to extend through an angle of at least 240 degrees around the body part. This may ensure that the structure may be worn around the body part without falling off.

The structure may have a size and sufficient stiffness for arranging the structure relatively tightly around the body part. This may imply that the carrier may be held in place in relation to the body part while allowing the carrier to be manually rotated if an adjustment of the position of the carrier is needed.

Thus, the carrier may be worn around the body part without being fully closed around the body part. This may be used for instance in a collar form factor to be worn around a neck. A carrier which is not fully closed may provide a more comfortable arrangement around the body part.

According to an embodiment, the processing unit is configured to determine a direction of adjustment of a position of the carrier for improving placement of the array of ultrasound transducers in relation to the anatomical part.

Thus, the system may be configured to provide feedback to a user for aiding positioning of the carrier in relation to the body part so as to provide a proper placement of the array of ultrasound transducers in relation to the anatomical part.

For instance, the processing unit may be configured to identify a location of a portion of the anatomical part in relation to ultrasound transducers at a periphery of the array. This implies that the entire anatomical part or only a small portion of the anatomical part is located in relation to the array. If the array of ultrasound transducers is further to be used for acquiring additional information of the anatomical part, the carrier may need to be re-positioned to ensure that the anatomical part is located in relation to a center of the array of ultrasound transducers. Hence, a larger portion of the anatomical part may be related to the array of ultrasound transducers providing an improved placement of the array of ultrasound transducers in relation to the anatomical part.

The system may comprise a feedback unit for providing a feedback signal to a user based on the direction of adjustment determined by the processing unit. Thus, the processing unit may output information to the feedback unit for presenting feedback to a user.

The feedback unit may be configured to output information in a manner that may be perceived by a user. Thus, the feedback unit may for instance be configured to output visible, audible, or tactile information. For instance, the feedback unit may comprise a display for providing display of visible information for instructing adjustment of the position. The feedback unit may however alternatively comprise one or more light sources, such as light emitting diodes (LEDs), that may be controlled to flash in dependence of the direction of adjustment. The feedback unit may comprise a loudspeaker that may be configured to output voice instructions for adjustment of position or to output beeping sounds in dependence of the direction of adjustment, such as providing different intervals of beeps for guiding placement of the array of ultrasound transducers in relation to the anatomical part. The feedback unit may comprise a vibrator configured to vibrate in dependence of the direction of adjustment, such as providing different intervals of vibrations for guiding placement of the array of ultrasound transducers in relation to the anatomical part.

According to an embodiment, the array of ultrasound transducers is a two-dimensional array having a size of at least 5 mm, such as at least 1 cm, such as at least 5 cm, in each dimension.

It should be realized that if the array is large, placement of the array of ultrasound transducer to enable locating the anatomical part is easy and requires low accuracy in correct placement. However, if the array is large, scanning of the entire array for acquiring pulse echo data may become time consuming. Thus, a trade-off between size and scanning time may need to be considered in a choice of size the array.

Thanks to the determining of the location of the anatomical part not requiring constructing an image, processing of acquired ultrasound data requires relatively low processing capacity, allowing a large array of ultrasound transducers to be used. The array of ultrasound transducers may be arranged in a square shape, wherein each side of the array is at least 5 mm, such as at least 1 cm, such as at least 5 cm. However, it should be realized that the array may for instance have a rectangular shape instead.

In some embodiments, the array of ultrasound transducers has a narrow size in one dimension, while having a large size in another perpendicular dimension. For instance, the array of ultrasound transducers may have a rectangular shape, with a narrow size in one dimension, while having a size of at least 5 mm, such as at least 1 cm, such as at least 5 cm, in the other dimension. In fact, in some embodiments, the array of ultrasound transducers may be a one-dimensional array with a single row of ultrasound transducers, wherein the row may have a size of at least 5 mm, such as at least 1 cm, such as at least 5 cm.

According to an embodiment, the processing unit is configured to analyze the pulse echo data in relation to the characteristic pattern of pulse echo representing the structure of a blood vessel.

Thus, the system may be used for localization of the blood vessel, such as an artery. The system may determine the location of the blood vessel based on the characteristic pattern corresponding to the structure of the blood vessel. Thus, the blood vessel may be identified without necessarily using information of dynamic behavior of the blood vessel, i.e., without using information relating to blood flow through the blood vessel. However, it should be realized that the dynamic behavior of the blood vessel may in some embodiments be used in combination with the information of the structure of the blood vessel for identifying the blood vessel.

The system being configured to provide localization of the blood vessel may be advantageously used for ensuring that the array of ultrasound transducers (and possibly other components of the system) is accurately positioned in relation to the blood vessel. This may ensure that the system may ensure that further measurements on the blood vessel for assessing cardiovascular health of the subject may be properly acquired. Thus, the system may facilitate robust and reliable cardiovascular health assessment, which need not even involve a skilled medical practitioner for placing the array of ultrasound transducers in relation to the blood vessel.

According to an embodiment, the processing unit is configured to analyze the pulse echo data in relation to the characteristic pattern including two amplitude peaks separated by a hypoechoic portion.

It is a realization that pulse echo data of the blood vessel provides a characteristic pattern relating to relatively strong reflections by walls of the blood vessel and a weak reflection, such as no reflection at all, from an interior of the blood vessel. In addition, the array of ultrasound transducers may be positioned in relation to a cross-section of a blood vessel. This implies that a transmitted pulse of ultrasound signal may first impinge on a first wall portion (anterior wall) closest to the ultrasound transducers, thereafter pass through the interior of the blood vessel, and impinge on a second wall portion (posterior wall) farther away from the ultrasound transducers. Hence, the pulse echo data from a blood vessel may comprise two peaks separated by a hypoechoic portion.

The wall portions may be separately identified based on amplitude peaks. However, it is a realization that processing pulse echo data in relation to two amplitude peaks separated by the hypoechoic portion may be a robust manner of identifying the blood vessel.

The processing unit may be configured to compare the pulse echo data to the characteristic pattern for identifying presence of the characteristic pattern. This may be based on digital signal processing.

However, according to an alternative embodiment, the characteristic pattern may be used for training a neural network such that the processing unit may use the neural network trained using the characteristic pattern for processing the pulse echo data. For instance, the characteristic pattern of the blood vessel may be used as a kernel for training the neural network.

According to an embodiment, the control unit is configured to control the selected subset to transmit and/or receive the ultrasound signals in plane wave mode or in focused mode.

The array of ultrasound signals may be used in a plane wave mode for determining information relating to a plane or volume extending from the array of ultrasound transducers into tissue of the subject. In the plane wave mode, a cross-section of transmitted ultrasound signals may be increasing into the tissue based on a divergence angle of the ultrasound signal transmitted from the transducers. Information of the entire plane at each depth into the tissue may be received in the pulse echo data.

The use of the plane wave mode may allow acquiring information from a relatively large volume of the tissue of the subject simultaneously.

The control unit may be configured to provide beam steering in order to control a direction of a plane wave of the ultrasound signals. This may be achieved using a plurality of ultrasound transducers as a phased array.

The array of ultrasound signals may be used in a focused mode for determining accurate information from a specific portion of the tissue. The focused mode may be achieved by providing a focus of transmitted ultrasound signals from a plurality of ultrasound transducers or by providing a focus of received ultrasound signals by a plurality of ultrasound transducers, such that the plurality of ultrasound signals defines a focus of an origin of the ultrasound signals received.

A penetration depth of the ultrasound signals may be dependent on the frequency of the ultrasound signals. Thus, a lower frequency may allow a larger penetration depth. On the other hand, a higher frequency may allow a higher spatial resolution for detecting features in the tissue.

The frequency of the transmitted ultrasound signals may thus be set in relation to a desired depth and resolution. For instance, the system may be configured to provide localization of aorta or arteries in an abdominal region requiring relatively large penetration depth. In such case, a center frequency of the ultrasound signals may for instance be tuned to 2-3 MHz. The system may also or alternatively be configured to provide localization of a carotid artery in the neck of the subject. In such case, a center frequency of the ultrasound signals may for instance be tuned to 7-8 MHz to provide high resolution at relatively lower penetration depth.

It should be realized that the system may be designed for a specific application. Thus, the ultrasound signals may use a pre-set frequency adapted to the specific application and there may be no need for providing tuning of the frequency.

However, in embodiments, the array of ultrasound transducers may comprise ultrasound transducers having different characteristics. For instance, a first part of the array may comprise ultrasound transducers adapted for transmitting ultrasound signals at a relatively high frequency and a second part of the array may comprise ultrasound transducers adapted for transmitting ultrasound signals at a relatively low frequency. Thus, the control unit may be configured to activate a desired part of the array in dependence of desired depth and resolution of the ultrasound signals. The first and second parts may be arranged in any manner in the array, such as being formed by separate areas in the array or in alternating arrangements of transducers belonging to the first part and transducers belonging to the second part.

According to another embodiment, the array of ultrasound transducers may be removably arranged on the carrier. This implies that the array of ultrasound transducers may be replaced by another array of ultrasound transducers having different characteristics. Thus, if the system needs to be adapted for output of a different frequency of the ultrasound signals, the array of ultrasound transducers may be replaced by another array that is suited for output of the desired frequency.

According to an embodiment, the subset of at least one ultrasound transducer is configured to, in the localization mode, transmit a plurality of pulses of ultrasound signals and to detect a plurality of reflected pulses, wherein the processing unit is configured to analyze the pulse echo data to identify a pulsatile change of the structure of the anatomical part for identifying that pulse echo data represents the anatomical part.

Thus, the system may be configured to use dynamic information for determining the location of the anatomical part. The dynamic information may be used in combination with static information relating to the structure of the anatomical part.

For instance, when the system is used for localization of a blood vessel, pulsatile changes of the structure of the blood vessel may be used for identifying the blood vessel. It should be realized that walls of the blood vessel slightly change position during propagation of a pulse wave through the blood vessel. The blood vessel is dilated and contracted during the pulse wave propagation. This may be identified by changes in position of the vessel walls being detected and may further be used for identifying that the pulse echo data represents a blood vessel.

It should be realized that the pulsatile changes may not necessarily be detected based on a frequency shift of the ultrasound signal caused by movement of the walls of the blood vessel or by blood flow in the blood vessel. Rather, the pulsatile changes may be determined based on a change in position of the walls with respect to different pulses in the plurality of pulses.

The processing unit may be configured to identify a pulsatile variation in the change of the structure corresponding to a change that is periodically occurring. A frequency of the pulsatile variation may be used for identifying the blood vessel.

According to an embodiment, the control unit is further configured to, based on the location of the anatomical part, activate one or more subsets of ultrasound transducers for controlling the one or more subsets to assume a spatial information acquisition mode, wherein each ultrasound transducer in the one or more subsets is configured to transmit one or more pulses of ultrasound signals into the subject and to detect the reflected one or more pulses for forming pulse echo data representing spatial information of the anatomical part.

Thus, the system may be used for determining further information of the anatomical part once the anatomical part has been localized. The array of ultrasound transducers may thus be used both for determining location of the anatomical part and for determining further information of the anatomical part, which may for instance be used for assessment of health of the anatomical part.

The spatial information acquisition mode may be used for determining detailed spatial information of the anatomical part after localization of the anatomical part. For instance, size or morphological parameters may be determined, which may be used for assessment of health of the anatomical part.

The system may be configured to use location information of the anatomical part to activate ultrasound transducers for transmitting targeted ultrasound signals for acquiring spatial information. Thus, spatial information may be acquired from a location of interest based on the location information acquired in localization of the anatomical part.

The system may be configured to determine a location of a blood vessel and further, in the spatial information acquisition mode of the subset(s) of ultrasound transducers acquire information for determining parameters relating to cardiovascular health. For instance, the system may be configured to determine parameters, such as diameter of a blood vessel or intima-media thickness. In addition, the spatial information may be used for determining presence of a condition, such as presence of stenosis or vascular dissection.

According to an embodiment, the control unit is further configured to, based on the location of the anatomical part, activate one or more subsets of ultrasound transducers for controlling the one or more subsets to assume a flow information acquisition mode, wherein each ultrasound transducer in the one or more subsets is configured to transmit ultrasound signals into the subject and to detect the reflected ultrasound signals for representing Doppler frequency and/or Doppler shift based on a flow in the anatomical part.

The ultrasound transducers may be able to assume both the spatial information acquisition mode and the flow information acquisition mode. Thus, both spatial information and flow information may be acquired. However, it should be realized that the ultrasound transducers may in some embodiments only be able to be used for acquiring spatial information or for acquiring flow information.

The flow information acquisition mode may be used for determining detailed information relating to a flow in the anatomical part after localization of the anatomical part. For instance, a velocity of the flow may be determined, which may be used for assessment of health of the anatomical part.

The system may be configured to use location information of the anatomical part to activate ultrasound transducers for transmitting targeted ultrasound signals for acquiring flow information. Thus, flow information may be acquired from a location of interest based on the location information acquired in localization of the anatomical part.

The system may be configured to determine a location of a blood vessel and further, in the flow information acquisition mode of the subset(s) of ultrasound transducers acquire flow information for determining parameters relating to cardiovascular health. The system may be configured to use location information of the blood vessel to activate ultrasound transducers for transmitting targeted ultrasound signals for acquiring flow information. For instance, the control unit may be configured to control the activated subset(s) of ultrasound transducers to output ultrasound signals at a desired angle in relation to the blood flow, and/or to direct the ultrasound signals to a target location, e.g., at a central position in the blood vessel or close to a vessel wall.

The system may be configured to use the flow information acquired in the flow information acquisition mode to determine one or more parameters, such as parameters relating to flow velocity or to waveforms of flow.

It should also be realized that flow information may in some embodiments be used for aiding localization of the anatomical part and/or confirming localization of the anatomical part. The flow information may provide information of blood flow but may also provide information of other flow of liquids in a body, such as flow in a urine tract and/or lymph system.

According to an embodiment, the system is further configured to acquire arterial information of an artery in the anatomical part, and wherein the processing unit is configured to process the arterial information for determining at least one parameter for characterization of the artery, such as the at least one parameter being pulse wave velocity.

The arterial information may provide important input for assessment of cardiovascular health. The at least one parameter may comprise a functional parameter representing a function of the artery. The at least one parameter may thus comprise a parameter for mechanical characterization of the artery. Parameters for mechanical characterization may provide information of stiffness of arteries. For instance, pulse wave velocity is a frequently used parameter for assessment of cardiovascular health.

The at least one parameter may also or alternatively comprise a structural parameter of the artery. The structural parameter may for instance be a wall thickness of the artery.

According to an embodiment, the system further comprises one or more additional sensors selected from the group of an electrocardiogram sensor, an inertial measurement unit, a blood pressure sensor, and a tonometer.

This implies that the system may use the array of ultrasound transducers in combination with other sensors for determining information relating to health of the anatomical part.

The one or more additional sensors may acquire information in addition to information acquired by the array of ultrasound transducers for assessment of health of the anatomical part. However, in some embodiments, only information acquired by the one or more additional sensors is used for assessment of health of the anatomical part.

The localization of the anatomical part provided by the array of ultrasound transducers may be used for guiding acquisition of further information by at least some of the additional sensors. Thus, the additional sensor(s) may be directed for acquiring information from the anatomical part based on the localization of the anatomical part.

According to an embodiment, the one or more additional sensors may be arranged on a separate carrier from the array of ultrasound transducers.

Additional sensor(s) arranged on a separate carrier may be used for determining information that is used in combination with information acquired by the array of ultrasound transducers for assessment of health of the anatomical part. Additional sensor(s) arranged on a separate carrier may not be localized in relation to the anatomical part based on the location information acquired by the array of ultrasound transducers, as a relation between the array of ultrasound transducers and the separate carrier may not be known.

However, the additional sensor(s) may not need to be accurately located in relation to the anatomical part for acquiring relevant information. For instance, a blood pressure sensor may be configured to acquire information of blood pressure in relation to a location of the vascular system which is different from a location monitored by the array of ultrasound transducers. In addition, thanks to the one or more additional sensors being arranged on a separate carrier, the additional sensor(s) may allow acquiring information from a different part of the body of the subject.

It should be realized that one or more additional sensors may also or alternatively be arranged on a common carrier with the array of ultrasound transducers. This may facilitate a simple placement of the additional sensor(s) and the array of ultrasound transducers in relation to the subject, as a single carrier may be arranged in relation to the subject. In addition, the one or more additional sensor(s) arranged on the common carrier with the array of ultrasound transducers may also be localized in relation to the anatomical part, so as to allow guiding of acquisition of information from the anatomical part by the additional sensor(s).

It should further be realized that in some embodiments, one or more additional sensors are arranged on a common carrier with the array of ultrasound transducers and one or more other additional sensors are arranged on a separate carrier.

According to an embodiment, the system is configured to detect at least one parameter for estimating blood pressure based on at least one parameter.

This facilitates acquisition of an important parameter for assessment of cardiovascular health.

According to an embodiment, the control unit is further configured to control the array of ultrasound transducers to transmit a targeted pulse based on the localization of the anatomical part in the subject, such as controlling the array of ultrasound transducers to transmit the targeted pulse for stimulating the anatomical part and/or controlling the array of ultrasound transducers to transmit the targeted pulse for mechanically characterizing tissue of the anatomical part.

Thus, the localization of the anatomical part in the subject may be used for controlling further use of the array of ultrasound transducers. The localization of the anatomical part may thus ensure that the targeted pulse is accurately directed to the anatomical part so as to ensure efficient stimulation and/or accurate detection of further information relating to the anatomical part.

According to a second aspect, there is provided a method for localization of an anatomical part in a subject, said method comprising: sequentially activating different selected subsets of at least one ultrasound transducer from an array of ultrasound transducers for controlling the subset to assume a localization mode, wherein each ultrasound transducer in the selected subset is configured to transmit one or more pulses of ultrasound signals into the subject and to detect the reflected one or more pulses for forming pulse echo data representing an ultrasound response from the subject for each pulse as a function of a time delay of the ultrasound response; analyzing the pulse echo data acquired by a plurality of selected subsets in relation to a characteristic pattern of pulse echo representing a structure of the anatomical part, and identifying one or more ultrasound transducers being located to acquire pulse echo data representing the anatomical part for determining a location of the anatomical part in the subject.

Effects and features of this second aspect are largely analogous to those described above in connection with the first aspect. Embodiments mentioned in relation to the first aspect are largely compatible with the second aspect.

The method enables localization of the anatomical part of the subject in a simple manner. The ultrasound transducers acquire pulse echo data from the subject. The pulse echo data may be directly received from the ultrasound transducers and there may be no need of complicated processing of signals detected by the ultrasound transducers for providing a representation of the anatomy of the subject in order to allow identification of the anatomical part. In particular, there is no need of constructing an image of the anatomy of the subject in order to allow the anatomical part to be identified in the image.

According to a third aspect, there is provided a system for assessment of an anatomical part in a subject, said system comprising:
an array of ultrasound transducers configured to transmit ultrasound signals into the subject and to detect reflected ultrasound signals from the subject;
a control unit configured to activate a selected subset of at least one ultrasound transducer from the array of ultrasound transducers for controlling the subset to assume a localization mode, wherein the control unit is further configured to sequentially activate different selected subsets from the array of ultrasound transducers;
a processing unit configured to analyze the pulse echo data acquired by a plurality of selected subsets for determining a location of the anatomical part in the subject;
wherein the control unit is further configured to, based on the determined location of the anatomical part, activate one or more subsets of ultrasound transducers for controlling the one or more subsets to assume an information acquisition mode, wherein the control unit is configured to set one or more parameters of the information acquisition mode in dependence of the determined location of the anatomical part.

Effects and features of this third aspect are largely analogous to those described above in connection with the first and second aspects. Embodiments mentioned in relation to the first and second aspects are largely compatible with the third aspect.

Thanks to using an array of ultrasound transducers, the system provides lenient requirements on placement of the array of ultrasound transducers in relation to the anatomical part of interest. Thus, even if the array of ultrasound transducers is not perfectly aligned with the anatomical part, at least some part of the array may be able to detect information from the anatomical part so as to allow localization of the anatomical part.

The system for localization of the anatomical part may provide a location of the anatomical part in relation to an arrangement of the array of ultrasound transducers.

The system may further be configured to control the ultrasound transducers to be used in an information acquisition mode, wherein the ultrasound transducers are controlled based on the information of the location of the anatomical part for ensuring that the information of the anatomical part may be properly acquired.

For instance, the control unit may select the subset of ultrasound transducers to be used in dependence of the location of the anatomical part. In addition, the control unit may control the subset of ultrasound transducers to transmit ultrasound towards the location of the anatomical part and/or to receive ultrasound from the location of the anatomical part.

The information acquisition mode may be a spatial information acquisition mode or a flow information acquisition mode. The control unit may be able to selectively control subset(s) of the ultrasound transducers to assume either the spatial information acquisition mode or the flow information acquisition mode. Thus, the system may have capability to use both the spatial information acquisition mode and the flow information acquisition mode. Alternatively, the system may only have capability to use one of the spatial information acquisition mode or the flow information acquisition mode.

According to an embodiment, the control unit is configured to, based on the location of the anatomical part, activate one or more subsets of ultrasound transducers for controlling the one or more subsets to assume a spatial information acquisition mode, wherein each ultrasound transducer in the one or more subsets is configured to transmit one or more pulses of ultrasound signals into the subject and to detect the reflected one or more pulses for forming pulse echo data representing spatial information of the anatomical part.

According to another embodiment, the control unit is configured to, based on the location of the anatomical part, activate one or more subsets of ultrasound transducers for controlling the one or more subsets to assume a flow information acquisition mode, wherein each ultrasound transducer in the one or more subsets is configured to transmit ultrasound signals into the subject and to detect the reflected ultrasound signals for representing Doppler frequency and/or Doppler shift based on a flow in the anatomical part.

### Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.
Fig. 1 is a schematic view of a system according to an embodiment.
Fig. 2 is a schematic view illustrating possible placements of a carrier of the system.
Figs 3a-c are schematic views illustrating activations of selected subsets of ultrasound transducers in an array.
Fig. 4a is a graph illustrating a characteristic pattern of pulse echo data from a blood vessel.
Fig. 4b is a schematic view illustrating localization of a blood vessel in relation to an array of ultrasound transducers.
Fig. 5 is a schematic view illustrating feedback for readjustment of a position of a carrier.
Fig. 6 is a schematic view illustrating acquisition of spatial information of a blood vessel based on localization of the blood vessel.
Figs 7a-b are schematic views illustrating arrangement of components of the system in a wearable device and in a combination of a wearable device and a companion device, respectively.
Fig. 8 is a schematic view illustrating functionality of the system.
Fig. 9 is a flow chart of a method according to an embodiment.

### Detailed description

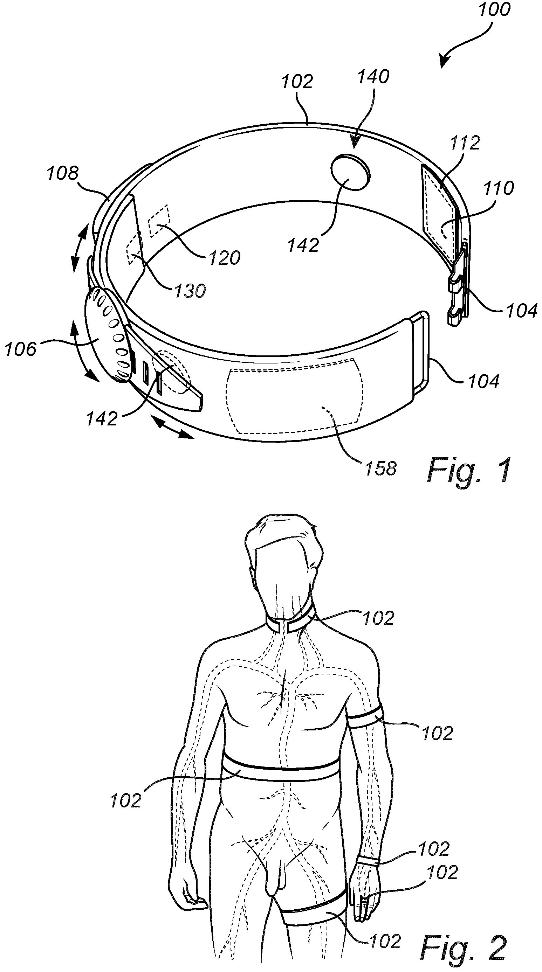

Referring now to Fig. 1, a system 100 for localization of an anatomical part in a subject will be described. The system 100 will be described below in relation to localization of a blood vessel and further to assessment of cardiovascular health. However, it should be realized that the system 100 may alternatively be used for localization of another anatomical part, possibly in combination with assessment of health of the anatomical part.

As shown in Fig. 1, the system 100 may comprise a carrier 102. An array of ultrasound transducers 110 may be arranged on the carrier 102. Thus, the carrier 102 may be designed for controlling placement of the array of ultrasound transducers 110 in relation to a blood vessel. However, it should be realized that the array of ultrasound transducers 110 need not necessarily be arranged on a carrier 102 and, in fact, different ultrasound transducers 110 or subsets of ultrasound transducers in the array may be arranged on different physical elements for providing individual placement of the ultrasound transducers 110 in relation to the anatomical part.

However, the use of the carrier 102 carrying the array of ultrasound transducers 110 provides a well-controlled placement of the ultrasound transducers 110 in relation to the anatomical part.

The carrier 102 may be adapted for placement in relation to a particular body part of the subject. The carrier 102 may thus provide an ergonomic design allowing the carrier 102 to be comfortably worn by the subject. The carrier 102 may further be configured to be adjustable in size in order to allow the carrier 102 to be properly placed in relation to the particular body part of the subject, regardless of size of the subject.

The carrier 102 may be configured to be adjustably positioned in relation to the body part. This implies that the carrier 102 may be re-positioned in order to adjust a relation between the array of ultrasound transducers 110 and the anatomical part. Thus, the system 100 does not require accurate knowledge of anatomy by a user in order to allow the array of ultrasound transducers 110 to be correctly arranged in relation to the anatomical part. Rather, if the carrier 102 is not properly placed, the carrier 102 may be re-positioned in order to properly position the carrier 102 in relation to the anatomical part. For instance, the carrier 102 may be placed by the subject, allowing self-use of the system 100 and hence no requirement of a skilled medical practitioner to be involved.

The carrier 102 may for instance be configured to be arranged firmly in position in relation to the body part of the subject, while still allowing the carrier 102 to be simply re-positioned. The carrier 102 may for instance comprise two mating elements on opposite sides along a longitudinal extension of the carrier 102. The mating elements 104 may thus be connected in order to arrange the carrier 102 around a body part. The carrier 102 may further comprise a size-adjustable element 106 for adjusting a longitudinal length of the carrier 102. This may be used for tightening the carrier 102 around the body part to ensure that the carrier 102 is firmly arranged in relation to the body part. Thus, in some embodiments, the carrier 102 may be configured to be strapped around the body part.

However, it should be realized that the carrier 102 may be arranged in different manners for allowing adjustable positioning of the carrier 102. For instance, the carrier 102 may be stretchable in order to allow the carrier 102 to fit tightly around a body part.

The carrier 102 may be used for adjusting circumferential position of the array of ultrasound transducers 110 in relation to the body part. Thus, adjusting of the position of the carrier 102 may involve rotating the carrier 102 in relation to the body part. However, adjusting of the position of the carrier 102 may also or alternatively involve moving the carrier 102 to a different location around the body part, such as moving the carrier 102 along an arm of the subject.

The carrier 102 may be differently designed in dependence of an anatomical part that is targeted. The carrier 102 may be flexible to allow the carrier 102 to be bent for placing the carrier 102 around the body part. However, it should be realized that the carrier 102 may in some embodiments be rigid. The carrier 102 may in such case comprise a hinge for allowing two rigid parts to be opened and closed for arranging the carrier 102 around the body part, completely or partially surrounding the body part.

According to an embodiment, the carrier 102 may comprise a shape memory property or a bias towards a particular shape of the carrier 102. Thus, the carrier 102 may for instance have a bent shape for fitting around the body part. The carrier 102 may be opened by applying a force on the carrier 102 for arranging the carrier 102 in relation to the body part, and upon release of the force, the carrier 102 may then assume the bent shape for placing the carrier 102 in position around the body part.

The carrier 102 may alternatively comprise a shape memory material which may be switched between two different shapes depending, e.g., on temperature of the material. This may be used for arranging the carrier 102 around the body part.

Given as non-limiting examples, the following form factors of the carrier 102 may be envisioned. Possible arrangements of carriers 102 around body parts is also illustrated in Fig. 2.

The carrier 102 may be implemented as a neck collar, which may be adjustable in circumference and possibly also adjustable in width. This may be used for localization of carotid arteries and/or jugular veins.

The carrier 102 may be implemented as a chest belt, which may be adjustable in circumference. This may be used for localization of aorta, pulmonary artery, and/or vena cava.

The carrier 102 may be implemented as an abdominal belt, which may be adjustable in circumference. This may be used for localization of abdominal aorta and/or vena cava.

The carrier 102 may be implemented as a thigh belt or thigh cuff, which may be adjustable in circumference. This may be used for localization of femoral artery and/or femoral vein.

The carrier 102 may be implemented as an ankle cuff, which may be adjustable in circumference. This may be used for localization of tibial artery and/or tibial vein.

The carrier 102 may be implemented as an arm belt or arm cuff, which may be adjustable in circumference. This may be used for localization of brachial artery and/or brachial vein.

The carrier 102 may be implemented as a wrist bracelet, which may be adjustable in circumference. This may be used for localization of radial artery and/or ulnar artery.

The carrier 102 may be implemented as a finger ring. This may be used for localization of digital artery.

The carrier 102 may be implemented as a wrap fitting, which may be adapted to be arranged for instance around the neck, upper arm, lower arm, thigh, or a finger of the subject.

One or more arrays of ultrasound transducers 110 may be arranged on the carrier 102. In addition, one or more additional sensors 140 may be arranged on the carrier 102 for enabling acquisition of further information relating to the anatomical part or generally relating to assessment of health of the subject. The additional sensors 140 may include two electrocardiogram electrodes 142 being arranged on the carrier 102, as illustrated in Fig. 1.

The array of ultrasound transducers 110 may be a large array configured to provide wide coverage of a surface area of the body part, such that the blood vessel of interest is likely to be located under the surface area in which the array of ultrasound transducers 110 is arranged. This facilitates placing the array of ultrasound transducers 110 such that the array of ultrasound transducers 110 are arranged in relation to the blood vessel for allowing transmitting ultrasound signals to the blood vessel and detecting ultrasound signals reflected from the blood vessel.

The array of ultrasound transducers 110 may be a one-dimensional array or may be a two-dimensional array. In addition, as mentioned above, more than one array of ultrasound transducers 110 may be arranged on the carrier 102. Hence, the arrays may be selectively used in order to find the array of ultrasound transducers 110 being placed in relation to the anatomical part and for localizing the anatomical part in relation to such array. It should also be realized that different arrays of ultrasound transducers 110 may be used for localizing different anatomical parts. For instance, different arrays may simultaneously be arranged in relation to different blood vessels for allowing localization of multiple blood vessels in one placement of the carrier 102.

The use of more than one array of ultrasound transducers 110 may allow each array to be smaller while allowing lenient requirements on placement of the carrier 102 in order for ultrasound transducers 110 to be properly arranged in relation to the anatomical part.

According to an embodiment, the array of ultrasound transducers 110 is a two-dimensional array having a size of at least 5 mm, such as at least 1 cm, such as at least 5 cm, in each dimension.

It should be realized that if the array is large, placement of the array of ultrasound transducer 110 to enable locating the anatomical part is easy and requires low accuracy in correct placement. However, if the array is large, scanning of the entire array for acquiring information from the array may become time consuming. Thus, a trade-off between size and scanning time may need to be considered in a choice of size the array. Also, the size of the array may also be chosen in view of addressability of subsets of ultrasound transducers 110 in the array.

The array of ultrasound transducers 110 may be arranged in a square shape, wherein each side of the array is at least 5 mm, such as at least 1 cm, such as at least 5 cm. However, it should be realized that the array may for instance have a rectangular shape instead.

In some embodiments, the array of ultrasound transducers 110 has a narrow size in one dimension, while having a large size in another perpendicular dimension. For instance, the array of ultrasound transducers 110 may have a rectangular shape, with a narrow size in one dimension, while having a size of at least 5 mm, such as at least 1 cm, such as at least 5 cm, in the other dimension. In fact, in some embodiments, the array of ultrasound transducers 110 may be a one-dimensional array with a single row of ultrasound transducers 110, wherein the row may have a size of at least 5 mm, such as at least 1 cm, such as at least 5 cm.

According to another embodiment, the array of ultrasound transducers 110 may be arranged in an annular configuration around the body part. The ultrasound transducers 110 may thus be considered to be arranged in relation to a polar coordinate system and control of the ultrasound transducers 110 may be provided in relation to the polar coordinate system.

The arrangement of a plurality of ultrasound transducers 110 in one or more one-dimensional linear arrays could provide high efficiency when limited information from the anatomical part, such as limited hemodynamic signals, are to be acquired. However, a large two-dimensional array of ultrasound transducers 110 may be preferred for enabling broad functionality of the system 100.

A desired pitch (i.e., distance between center points of adjacent ultrasound transducers 110 in the array) may be related to a frequency of the ultrasound signals. Typically, a desired pitch may be half of a wavelength of the ultrasound signal, i.e., λ / 2 = (v / f_{c}) / 2, where v is speed of sound (in tissue typically 1540 m/s), and f_{c} is a center frequency of the ultrasound signal. The pitch may be larger than half of the wavelength but in some embodiments the pitch may not be larger than 0.75*wavelength.

The center frequency of the ultrasound signal may depend on the anatomical part to be localized. However, the center frequency may typically be in a range of 2-10 MHz. This implies that a desired pitch may be in order of 75 - 400 µm.

The number of ultrasound transducers 110 in the array depends on the pitch and the size of the array. It should be realized that the number of ultrasound transducers 110 in each dimension (i.e., along a row or column) may for instance be in a range of 10 - 1500.

The array of ultrasound transducers 110 may be configured to provide a redundancy of transducers. Thus, not all ultrasound transducers 110 may be needed for properly transmitting ultrasound signals to and receiving ultrasound signals from the anatomical part. Moreover, as discussed below, only one or a few ultrasound transducers 110 may be used at a time to ensure power efficient use of the ultrasound transducers 110.

The array of ultrasound transducers 110 may be mechanically integrated into the carrier 102 in different ways. For instance, the ultrasound transducers 110 may be spring-loaded to ensure appropriate contact pressure allowing a good comfort to the subject and high-quality signal acquisition.

The array of ultrasound transducers 110 may be arranged on the carrier 102 under an acoustically transparent protection layer (e.g., made from silicone). On top of the protective layer, the array of ultrasound transducers 110 may be covered with a customized (possibly disposable) medium acting as acoustic impedance matching layer, e.g., a hydrogel pad. The carrier 102 may comprise an enclosure defining a recess in which the acoustic impedance matching layer may be received. This may ensure intuitive fitting of the acoustic impedance matching layer on the carrier 102.

In addition, the system 100 may comprise a thin acoustic impedance matching layer, which may be arranged between the array of ultrasound transducers and the protective layer. The acoustic impedance matching layer may have a thickness of quarter of a wavelength of the ultrasound transmitted and received by the ultrasound transducers 110.

Further, the system 100 may comprise an acoustic backing layer, which may be arranged on a side of the array of ultrasound transducers opposite to a direction of ultrasound to be transmitted into the subject and received from the subject. The acoustic backing layer may ensure directivity of the transmitted ultrasound.

The carrier 102 may be adapted for a specific application corresponding to a specific placement of the carrier 102 in relation to a particular body part. The array of ultrasound transducers 110 may further be adapted for the specific application. For instance, the array of ultrasound transducers 110 may have different physical properties depending on the specific application. This may ensure that the array of ultrasound transducers 110 is adapted to provide high signal quality for transmission and reception of ultrasound signals.

For instance, the carrier 102 in form of a neck collar may be adapted for use in localization of carotid artery, which is superficial and hence close to a skin surface on which the array of ultrasound transducers 110 is placed. In this application, the ultrasound transducers 110 may be designed to be tuned to a center frequency of ultrasound signals being 7-8 MHz. This may provide a high axial resolution along an axis extending into tissue from the array of ultrasound transducers 110. The array of ultrasound transducers 110 may further be designed with a corresponding narrow pitch and small area of each of the ultrasound transducers 110.

In contrast, the carrier 102 in form of an abdominal belt may be adapted for use in localization of aorta, which may be arranged relatively deep into tissue (compared to the superficial placement of the carotid artery). In this application, the ultrasound transducers 110 may be designed to be tuned to a center frequency of ultrasound signals being 3-5 MHz. This may provide a relatively good axial resolution, while allowing a long penetration depth into tissue. The array of ultrasound transducers 110 may further be designed with a corresponding wider pitch and larger area of each of the ultrasound transducers 110 compared to the case of the neck collar.

A region in which the array of ultrasound transducers 110 is arranged may in some embodiments be rigid. This may be used in combination with an acoustic impedance matching layer allowing the array to be arranged conforming to a shape of the body part. This may for instance be used when the array of ultrasound transducers 110 is to be arranged in relation to a relatively planar surface of the body part, such as when the array of ultrasound transducers 110 is to be arranged on a torso or abdomen of the subject.

Alternatively, the array of ultrasound transducers 110 may be arranged in a flexible manner in at least one dimension to allow the array to conform with the anatomic structure. This may be achieved by the carrier 102, on which the array of ultrasound transducers 110 is arranged, being flexible. In this case, electrical (resistive / capacitive) or optical (fiber bend) flex sensor(s) may be arranged on the carrier 102 allowing sensing of radius curvature and position of the ultrasound transducers 110.

The system 100 further comprises a control unit 120. The control unit 120 may be configured to control transmission of ultrasound signals and reception of ultrasound signals by the array of ultrasound transducers 110. The control unit 120 may for instance be configured to control which ultrasound transducers 110 that are active for transmission and/or reception of ultrasound signals at a specific point in time.

The system 100 further comprises a processing unit 130. The processing unit 130 may be configured to process data acquired by the ultrasound transducers 110. The processing unit 130 may further be configured to process data acquired by additional sensors 140. The processing unit 130 may further be configured to provide information to the control unit 120 for providing input for control by the control unit 120 based on the data received by the processing unit 130.

As illustrated in Fig. 1, the system 100 may comprise a casing 108 arranged on the carrier 102. The casing 108 may provide protection for electronic components of the system 100 arranged on the carrier 102. The control unit 120 and the processing unit 130 may be arranged in the casing 108.

In addition, other components may be arranged in the casing 108 or may be separately arranged on the carrier 102. For instance, a power source, such as a battery, may be arranged in the casing 108. Also, memory and other electronic components may be arranged in the casing 108. The casing 108 may for instance also include a user interface for presenting information to a user and/or receiving user input. Thus, a display may be integrated in the casing 108.

The control unit 120 and the processing unit 130 may be commonly implemented in the general-purpose processing unit, such as a central processing unit (CPU), which may execute instructions of one or more computer programs in order to implement respective functionalities of the control unit 120 and the processing unit 130. Thus, the control unit 120 and the processing unit 130 may be implemented by different software modules or different execution threads executed by the general-purpose processing unit. However, it should be realized that the control unit 120 and the processing unit 130 may be separately implemented.

The control unit 120 may be implemented in a general-purpose processing unit. The control unit 120 may alternatively be implemented as firmware arranged e.g., in an embedded system, or as a specifically designed processing unit, such as an Application-Specific Integrated Circuit (ASIC) or a Field-Programmable Gate Array (FPGA).

Similarly, the processing unit 130 may be implemented in a general-purpose processing unit. The processing unit 130 may alternatively be implemented as firmware arranged e.g., in an embedded system, or as a specifically designed processing unit, such as an ASIC or an FPGA.

It should further be realized that at least the processing unit 130 but possibly also the control unit 120 may be arranged in a separate physical housing not integrated with the carrier 102.

For instance, the control unit 120 may be arranged in the casing 108 on the carrier 102, while the processing unit 130 may be arranged in a separate housing from the control unit 120 and may be configured to communicate with the control unit 120 through wired or wireless communication. The processing unit 130 may, e.g., be arranged in vicinity to the control unit 120 such as in a wearable or portable unit that the subject may be wearing, such as in a mobile phone or a smart watch. However, the processing unit 130 need not even be arranged in vicinity to the control unit 120 or the array of ultrasound transducers 110 but may rather be arranged anywhere, such as in a server "in the cloud".

It should also be realized that the control unit 120 may be similarly arranged separately from the array of ultrasound transducers 110. In addition, functionalities of the control unit 120 and/or the processing unit 130 may be distributed between more than one physical entity.

The use of the system 100 will now be described in further detail. As described below, the system 100 allows localization of an anatomical part, such as a blood vessel of interest based on simple and automated processing of data acquired by the array of ultrasound sensors 110. In particular, no construction of images is required for localization of the anatomical part. The system 100 may further provide fully automated extraction of parameters relating to assessment of health of the anatomical part, such as providing extraction of diameter and flow waveforms for computation of cardiovascular health parameters. Thanks to the simple positioning of the array of ultrasound transducers 110 in relation to the blood vessel and the localization of the blood vessel in relation to the array of ultrasound transducers 110, the system 100 ensures proper placement of the array of ultrasound transducers 110 for further ensuring reliable automated determination of parameters relating to cardiovascular health.

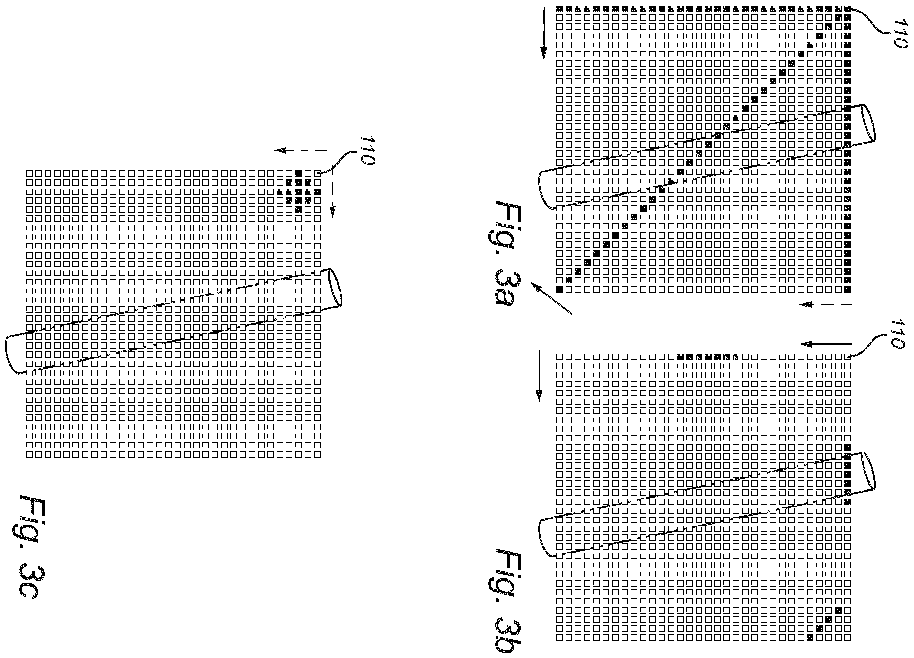

Initially, localization of a blood vessel will be discussed in relation to Figs 3a-c.

Thus, the carrier 102 may initially be placed in relation to a body part such that the array of ultrasound transducers 110 is arranged on the body part at an expected site of a location of the blood vessel. Thanks to the array of ultrasound transducers 110 being relatively large, there is a large likelihood that at least some part of the array of ultrasound transducers 110 is arranged so as to be able to transmit ultrasound signals to and receive ultrasound signals from the blood vessel.

The carrier 102 may also be designed to facilitate placement of the carrier 102 in such relation to the body part that the array of ultrasound transducers 110 is likely to be arranged such that some part of the array of ultrasound transducers 110 is arranged so as to be able to transmit ultrasound signals to and receive ultrasound signals from the blood vessel.

The control unit 120 is configured to activate a selected subset of at least one ultrasound transducer from the array of ultrasound transducers 110 for controlling the at least one ultrasound transducer 110 in the subset to assume a localization mode.

The at least one ultrasound transducer 110 in the selected subset is thus configured to transmit one or more pulses of ultrasound signals into the subject and to detect the reflected one or more pulses for forming pulse echo data. The pulse echo data represents an ultrasound response from the subject for each pulse as a function of a time delay of the ultrasound response.

Thanks to the control unit 120 activating a (small) subset of ultrasound transducers 110 at a time (illustrated by black squares in Figs 3a-c), each step of activating a subset requires small power consumption and produces a small amount of data to be processed. Thus, a limited number of ultrasound transducers 110 may be activated at a time, the limited number being a small fraction of the overall number of ultrasound transducers 110 in the array. The control unit 120 may be configured to address the ultrasound transducers 110 to be activated using a multiplexing scheme.

Figs 3a-c illustrate different manners of selecting the ultrasound transducers 110 of a subset. In Figs 3a-c, the array of ultrasound transducers 110 is illustrated as a grid overlying the blood vessel. The selected subset of ultrasound transducers 110 may be configured to transmit ultrasound signals into a cross-section of tissue extending along an axis perpendicular to the array of ultrasound transducers 110.

As shown in Fig. 3a, each selected subset of ultrasound transducers 110 may form a whole row, a whole column, or a whole diagonal in the array of ultrasound transducers 110.

As shown in Fig. 3b, according to an alternative, each selected subset of ultrasound transducers 110 may form a partial row, a partial column, or a partial diagonal in the array of ultrasound transducers 110.

As shown in Fig. 3c, each selected subset of ultrasound transducers 110 may form a two-dimensional sub-array in the array of ultrasound transducers 110.

The control unit 120 may be configured to continue sequentially activate different selected subsets from the array of ultrasound transducers 110 according to the set shape of subsets as exemplified above. This may be performed in order to scan the entire array along arrows indicated in Figs 3a-c. It should be realized that the selection of subsets may use an iterative strategy for finding the blood vessel. Once the blood vessel is found, the sequential selection of subsets may be terminated or continued using information of location of the blood vessel for mapping an extension of the blood vessel in relation to the array of ultrasound transducers 110.

The control unit 120 may be configured to control the selected subset to transmit and/or receive the ultrasound signals in plane wave mode or in focused mode.

The array of ultrasound signals may be used in a plane wave mode for determining information relating to a plane or volume extending from the array of ultrasound transducers 110 into tissue of the subject along an axis perpendicular to the array of ultrasound transducers 110. A certain aperture size may be needed for an acoustic wavefront to properly build up and reach a certain depth into the tissue. Therefore, the subset preferably comprises a plurality of ultrasound transducers 110.

Each ultrasound transducer 110 in the subset may be configured to determine respective pulse echo data corresponding to ultrasound responses along a line extending from the ultrasound transducer 110 into the tissue along the axis perpendicular to the array of ultrasound transducers 110. Thus, in the plane wave mode of transmitting and receiving ultrasound signals, an entire plane or volume corresponding to the ultrasound transducers 110 in the array may be examined for detection of the blood vessel.

The pulse echo data from a single ultrasound transducer 110 may be referred to as amplitude-mode (A-mode) ultrasound detection. Thus, when transmitting and receiving in plane wave mode, the A-mode signal from each ultrasound transducer 110 may be acquired providing a representation of acoustic responses along lines extending from the respective ultrasound transducer 110 into the tissue along the axis perpendicular to the array of ultrasound transducers 110.

The use of the plane wave mode may thus allow acquiring information from a relatively large volume of the tissue of the subject simultaneously.

The control unit 120 may also be configured to provide beam steering in order to control a direction of a plane wave of the ultrasound signals. This may be achieved using a plurality of ultrasound transducers as a phased array.

The array of ultrasound signals 110 may alternatively be used in a focused mode for determining accurate information from a specific portion of the tissue. The focused mode may be achieved by providing a focus of transmitted ultrasound signals from a plurality of ultrasound transducers 110 or by providing a focus of received ultrasound signals by a plurality of ultrasound transducers 110, such that the plurality of ultrasound signals defines a focus of an origin of the ultrasound signals received.

For instance, ultrasound transducers 110 in a row or partial row of the array may be used for forming a focused transmitted ultrasound signal. Then, a single ultrasound transducer, such as a centrally placed ultrasound transducer in the subset, may be used for receiving the ultrasound signal forming an A-mode signal. This may provide information of ultrasound response based on a focused ultrasound signal.

However, it should be realized that pulse echo data from a plurality of ultrasound transducers 110 may alternatively be combined. For instance, in the focused mode, an average of A-mode signals from a plurality of ultrasound transducers 110 may be formed, which may allow identifying the blood vessel using a higher signal-to-noise ratio.

It should further be realized that the selected subset may be used for transmitting and receiving a plurality of pulses. For instance, the control unit 120 may be configured to switch from a first subset to a next subset at a rate of 500 Hz, i.e., a switch to a next subset is made every 2 milliseconds. However, an acoustic pulse roundtrip is in order of microseconds. This implies that pulse echo data for a plurality of pulses may be determined. Hence, a change of A-mode pulse echo data over time may be determined, corresponding to motion-mode (M-mode) ultrasound detection. The detection of the blood vessel may be improved in the M-mode detection over A-mode detection. The M-mode may be used for detecting changes of the blood vessel over time, such as changes in position of walls due to arterial pulsations.

It should also be realized that transmitting and receiving ultrasound signals using a relatively large subset of ultrasound transducers 110 may imply that only some of the ultrasound transducers 110 receive pulse echo data corresponding to the blood vessel. The control unit 120 may be configured to initially activate selected subsets for coarse detection of the blood vessel and thereafter use refined detection using smaller subsets. For instance, the control unit 120 may initially control the selected subset of ultrasound transducers 110 to use a plane wave mode and thereafter control another selected subset of ultrasound transducers 110 for refined localization of the blood vessel to use a focused mode.

A sequential selection of different subsets of ultrasound transducers 110 may thus be used for finding the ultrasound transducers 110 arranged in relation to the blood vessel such that a line perpendicular to the array of ultrasound transducers 110 crosses both the blood vessel and the ultrasound transducer 110. The identification of such ultrasound transducers 110 allows localizing the blood vessel.

The blood vessel may thus be localized in relation to positions of ultrasound transducers 110 within the array of ultrasound transducers 110 being identified to detect pulse echo data representing the blood vessel. Thus, a lateral arrangement of the blood vessel in relation to the array of ultrasound transducers 110 may be determined. In addition, an axial position along an axis extending into tissue from the array of ultrasound transducers 110 may also be determined. The localization of the axial position may require use of focused mode for providing determination of the axial position with high resolution.

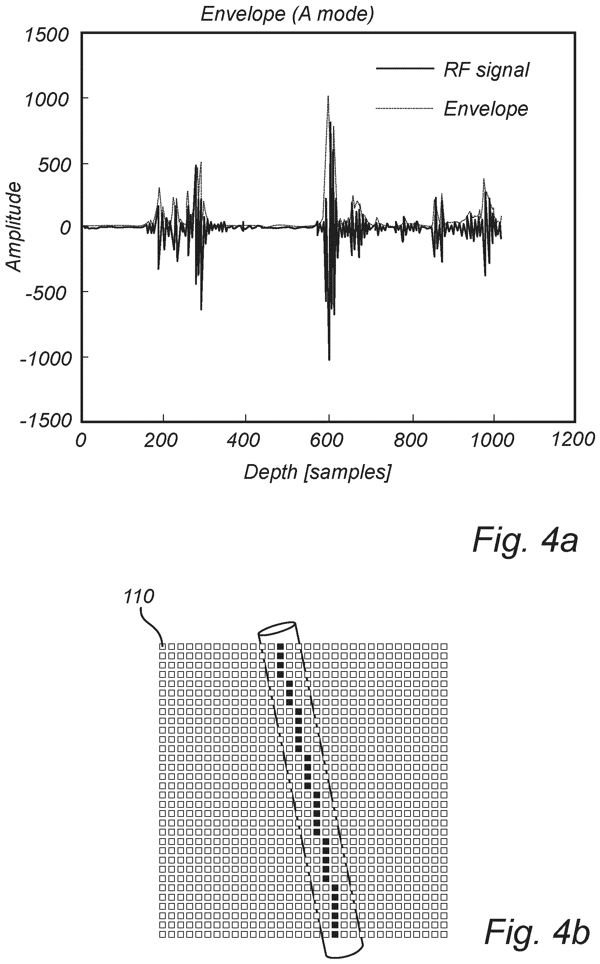

Referring now to Figs 4a-b, the processing unit 130 is configured to analyze the pulse echo data acquired by a plurality of selected subsets in relation to a characteristic pattern of pulse echo representing a structure of the anatomical part. The processing unit 130 is further configured to identify one or more ultrasound transducers 110 being located to acquire pulse echo data representing the anatomical part for determining the location of the anatomical part.

The processing unit 130 may be configured to perform pattern identification on the received pulse echo data, which may be represented in A-mode.

The processing unit 130 may be configured to analyze the pulse echo data in relation to the characteristic pattern of pulse echo representing the structure of a blood vessel. The characteristic pattern of a blood vessel is illustrated in Fig. 4a. The characteristic pattern of a cross-section of the blood vessel may be defined by a very low amplitude response from inside the vessel (due to intrinsic hypoechogenic character), bounded by two high amplitude peaks indicating the reflected echoes from the anterior and posterior walls of the blood vessel.

The A-mode signals may be analyzed to locate the exact anterior and posterior vessel walls using for instance an approach based on digital signal processing (DSP), wherein the pulse echo data is processed in relation to the characteristic pattern.

The A-mode signals may alternatively or additionally be analyzed to locate the exact anterior and posterior vessel walls using an approach based on artificial intelligence. For instance, a neural network architecture may be used for processing the pulse echo data, wherein the neural network architecture is trained to locate the anatomical part of interest based on training data including the characteristic pattern.

As illustrated in Fig. 4b, the blood vessel may be localized with relation to the array of ultrasound transducers 110. In Fig. 4b, positions in the plane of the ultrasound transducers 110 corresponding to the location of the blood vessel are indicated by black squares.

As mentioned above, the subset of ultrasound transducers 110 may be configured to transmit and receive a plurality of pulses of ultrasound signals. The processing unit 130 may then be configured to further analyze the pulse echo data to identify a pulsatile change of the structure of the blood vessel for identifying that pulse echo data represents the blood vessel. Such pulsatile information may thus be used for providing further reliability in localizing the blood vessel.

Advanced identification of a pulsatile artery or distinguishing non-pulsatile vein from a pulsatile artery may later be reinforced by extracting dynamic information of the pulsatile waveform, which can also be matched with characteristic patterns of certain types of blood vessels. In addition, flow information, such as speed of flow may be used for identifying certain types of blood vessels.

The controlling of sequential activation of subsets of ultrasound transducers 110 and associated processing of the acquired pulse echo data may output a location of the blood vessel in three dimensions (x, y, z coordinates). In particular, locations of anterior and posterior walls may be output.

The coordinates may be saved and forwarded to a location indicator. The location indicator may provide control for further actions by the system in relation to the blood vessel, such as further acquisition of information relating to the blood vessel. The location indicator may be seen as forming part of the processing unit 130, possibly as a separate software module or execution thread.

It should be realized that the dynamic motion character of the blood vessel (particularly for pulsatile arteries) implies that the locations of the walls of the blood vessel are obtained with certain tolerances. A more accurate detection may be provided using an ECG-gated approach in which the ultrasound array activation and pattern identification is always set to occur in the same phase of the cardiac cycle (ideal: end-diastolic).

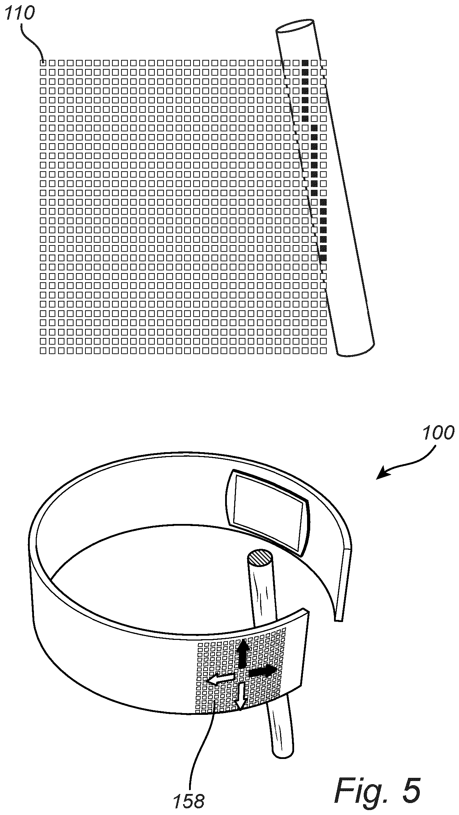

Referring now to Fig. 5, the location indicator may determine a quality of the vessel identification. The location indicator may provide feedback to a user to reposition or readjust the carrier 102. Feedback could simply be given by colored LEDs in form of arrows, or by a display acting as a window showing the alignment from a top view, supported by a surrounding color indicator.

If the location is optimal in relation to the array of ultrasound transducers 110, the blood vessel is maximized in the field of view of the array. This may ensure a minimum distance between two sub-arrays to be used for pulse wave velocity assessment.

If the array of ultrasound transducers 110 is not sufficiently well positioned in relation to the blood vessel for properly localizing the blood vessel or for allowing further acquisition of information to be reliably acquired, the carrier may need repositioning and/or size-adjustment.

The location indicator may thus determine a desired direction of adjustment of a position of the carrier 102 for improving placement of the array of ultrasound transducers 110 in relation to the anatomical part. Such information may be output as feedback to the user via a user interface 158, as shown in Fig. 5.

It should also be realized that the user interface 158, such as a display, e.g., a display acting as a window showing the alignment from a top view, could be used both for presenting feedback to the user but also to present other information (e.g., images) to a user and/or receiving user input.

The system 100 may be configured to acquire further information relating to the cardiovascular health once the blood vessel has been localized. The system 100 may be configured to provide monitoring of cardiovascular health such that the system 100 may continuously or intermittently acquire information for assessment of cardiovascular health over a long period of time. Thus, the carrier 102 with the array of ultrasound transducers 110 may be worn by the subject during a long period of time.

The system 100 may further be configured to provide re-identification of the blood vessel, i.e., to determine any update or change of the location of the blood vessel in relation to the array of ultrasound transducers 110. Artery re-identification may occur either at fixed timing intervals or be triggered event-based, such as being triggered if the characteristic pattern is no longer present in pulse echo data (e.g., as determined by amplitude thresholds required for peaks of the arterial walls).

The system 100 is configured to provide localization of an anatomical part, such as a blood vessel, as described above. The system 100 may further be configured to activate acquisition of spatial information for acquiring information relating to health of the anatomical part. The system 100 may be configured to control the acquisition of spatial information based on the determined location of the anatomical part. The system 100 may also or alternatively be configured to activate acquisition of flow information for acquiring information relating to health of the anatomical part. The system 100 may be configured to control the acquisition of spatial information based on the determined location of the anatomical part.

Thus, the control unit 120 may be further configured to, based on the location of the anatomical part, activate one or more subsets of ultrasound transducers 110 for controlling the one or more subsets to assume a spatial information acquisition mode. Each ultrasound transducer 110 in the one or more subsets is configured to transmit one or more pulses of ultrasound signals into the subject and to detect the reflected one or more pulses for forming pulse echo data representing spatial information of the anatomical part.

In addition, or alternatively, the control unit 120 may be further configured to, based on the location of the anatomical part, activate one or more subsets of ultrasound transducers 110 for controlling the one or more subsets to assume a flow information acquisition mode. Each ultrasound transducer 110 in the one or more subsets is configured to transmit ultrasound signals into the subject and to detect the reflected ultrasound signals for representing Doppler frequency and/or Doppler shift based on a flow in the anatomical part.

The control unit 120 may be configured to control spatial information acquisition and flow information acquisition in a time-interleaved manner to alternatingly perform spatial information acquisition and flow information acquisition. This may allow quasi-simultaneous acquisition of spatial and flow information, which may also be referred to as duplex scan.

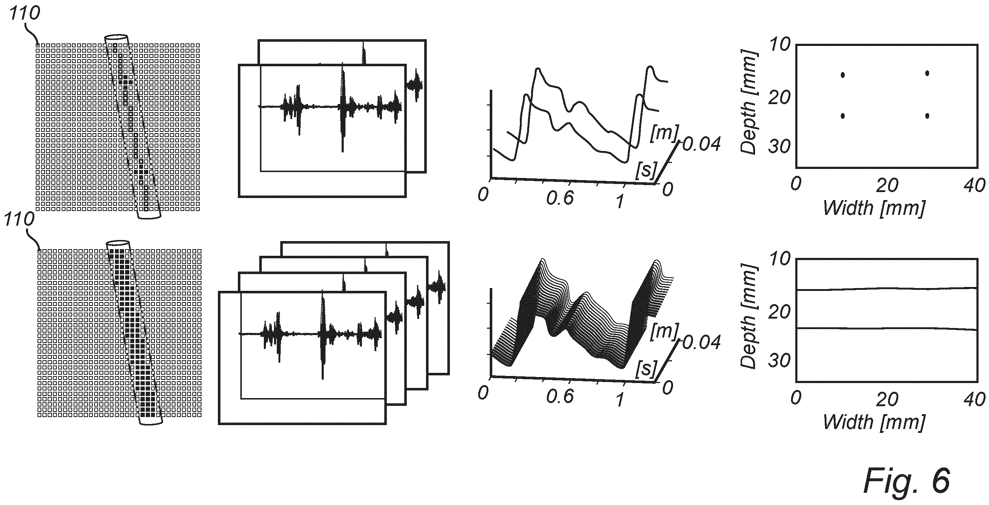

Referring now to Fig. 6, spatial information acquisition will be discussed in further detail.

The control unit 120 may be configured to activate at least one, preferably at least two sub-arrays of ultrasound transducers 110 at distinct locations in the array, to acquire spatial information that can be processed to structural and functional cardiovascular parameters. In an upper portion of Fig. 6, activation of two sub-arrays is illustrated as indicated by black squares, within previously identified blood vessel locations indicated by white-within-black squares, whereas in a lower portion of Fig. 6, activation of a large number of sub-arrays extending along the blood vessel is illustrated as indicated by black squares, covering all previously identified blood vessel locations.

Thus, the control unit 120 may be configured to, based on the obtained location information, as long as it remains valid, activate one or more sub-arrays simultaneously (at adequately high pulse repetition frequency) to obtain spatial information.

Similar to the discussion in relation to Figs 3a-c above, the sub-arrays can be sequentially activated.

The sequential activation may involve whole row-wise, column-wise, or diagonal scans. The ultrasound transducers 110 of the sub-array may be controlled to a plane wave mode or (transmit and/or receive) focused mode.

The sequential activation may alternatively involve partial row-wise, column-wise, or diagonal scans. The ultrasound transducers 110 of the sub-array may be controlled to a plane wave mode or (transmit and/or receive) focused mode.

The sequential activation may alternatively involve scanning of two-dimensional sub-arrays. The ultrasound transducers 110 of the sub-array may be controlled to a plane wave mode or (transmit and/or receive) focused mode.

It should be realized that thanks to the location of the blood vessel being known, scans may not need to be performed over the entire array of ultrasound transducers 110. This also implies that the system 100 may allow more computer intensive processing, since the processing may not need to be performed for data from the entire array of ultrasound transducers 110. In the leftmost part of Fig. 6, sub-arrays being activated are illustrated.

Simultaneous activation of at least two sub-arrays facilitates automated acquisition of at least two A-mode scanlines and corresponding M-mode representations. The processing unit 130 may be configured to process the data from each scanline, and may be configured to automatically extract high-quality vascular diameter and distension waveforms and/or to compute various functional parameters. The A-mode data and corresponding M-mode representations are illustrated in a middle portion of Fig. 6. It should however be noted that for clarity, only part of the data is shown in Fig. 6. Thus, while the upper row of Fig. 6 shows two sub-arrays being used and show two different sets of pulse echo data being acquired with two corresponding time-series waveforms. Further, the lower row only shows 4 of many A-mode signals indicating that more scanlines are used in the lower row setting to the upper row setting.

It should be further realized that the activation of multiple adjacent elements or sub-arrays not only facilitates multiple A-modes to be acquired, but also enables echo acquisition along a spatial plane. This may be used by the processing unit 130 to construct a full brightness mode (B-mode) image to enable structural information from the blood vessel to be extracted from the B-mode image.

The spatial information acquisition may be used for determining vascular diameter waveforms and/or high-resolution B-mode images.

It should further be realized that scanning of two-dimensional sub-arrays of the ultrasound transducers 110 may even be used for acquiring three-dimensional information of the anatomical part, or even four-dimensional information by continuously updating the three-dimensional information over time.

The control unit 120 may also or alternatively be configured to control the array of ultrasound transducers 110 to activate at least one sub-array of ultrasound transducers 110 to acquire blood flow information. The control unit 120 may be configured to activate the sub-array of ultrasound transducers 110 in a continuous wave Doppler mode or a pulsed wave Doppler mode.

Thus, the control unit 120 may be configured to, based on the obtained location identification, as long as it remains valid, activate one or more sub-arrays, wherein the ultrasound transducers 110 in the sub-array(s) extend in parallel with a longitudinal direction of the blood vessel, to transmit and receive a continuous or pulsed ultrasound wave, targeting a lumen of the blood vessel under a specified angle. The angle of the transmitted ultrasound may be provided by controlling delays of the ultrasound signals transmitted by the ultrasound transducers 110 in the sub-array(s). The ultrasound transducers may be configured to obtain at least one Doppler frequency and/or Doppler shift based on blood flow in the blood vessel.

The control unit 120 may be configured to control the ultrasound transducers 110 to target multiple locations within the identified blood vessel, to obtain multi-site flow information, e.g., mid lumen and towards edges at the vessel wall.

The flow information acquisition may be used for determining blood flow waveforms.

In addition or alternatively to the spatial acquisition information and the flow acquisition information, the system 100 may be configured to use the localization of the anatomical part in the subject for controlling one or more targeted ultrasound pulses.

Thus, the control unit 120 may be further configured to control the array of ultrasound transducers 110 to transmit a targeted pulse based on the localization of the anatomical part in the subject. This may be used for controlling the array of ultrasound transducers 110 to transmit the targeted pulse for stimulating the anatomical part and/or controlling the array of ultrasound transducers 110 to transmit the targeted pulse for mechanically characterizing tissue of the anatomical part.

When using a targeted pulse for stimulating the anatomical part, in contrast to acquiring of pulse echo data, the control unit 120 may be configured to control a sub-array of ultrasound transducers 110 to be set in a transmit-only mode and typically be using more powerful output of ultrasound signals compared to ultrasound signals used for acquiring pulse echo data. It should further be realized that a different array of ultrasound transducers may thus be used for output of the powerful ultrasound signals for stimulation.

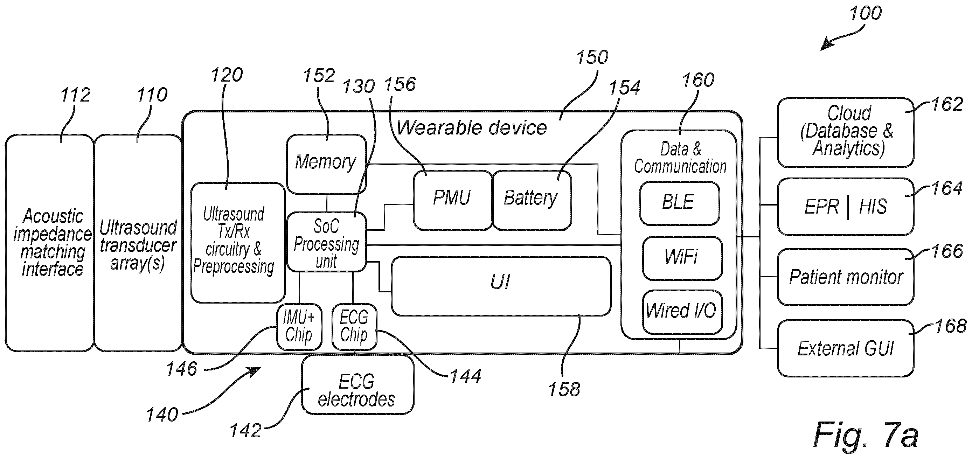

Referring now to Figs 7a-b, architecture of the system 100 according to two different embodiments will be described. It should be realized that further alternative arrangements of functional blocks in different physical entities may be provided, and the present description is not limited to the examples of the illustrated embodiments.

As illustrated in Fig. 7a, the system 100 may comprise a wearable device 150, which may incorporate most of the features discussed above. The wearable device 150 may include the carrier 102 as discussed above providing a possibility of arranging the wearable device 150 on the subject.

The wearable device 150 may comprise the array(s) of ultrasound transducers 110 which may be provided with a (disposable) acoustic impedance matching interface112 to the subject.

The wearable device 150 may further comprise electronic components providing functionality of the system 100. As illustrated in Fig. 7a, the wearable device 150 may incorporate ultrasound transmitting and receiving circuitry implementing the control unit 120 discussed above. This circuitry may also provide pre-processing of received ultrasound signals.

The wearable device 150 may further comprise a processing unit 130 in form of a system on a chip (SoC) processing unit. The SoC processing unit may implement the functionality of the processing unit 130 discussed above.

The wearable device 150 may further comprise memory 152 storing parameters and/or software to be executed by the SoC processing unit 130. The memory 152 may also be used for storing, at least temporarily, any information acquired by sensors of the system 100.

The wearable device 150 may further comprise a battery 154 and a power management unit (PMU) 156 for controlling powering of components of the system 100.

The wearable device 150 may further comprise a user interface 158 which may provide a simple interaction with a user, e.g., through buttons, LEDs, display, speakers and/or vibrotactile elements.

The wearable device 150 may also incorporate additional sensors 140 which may be arranged on the carrier 102 for enabling acquisition of further information relating to the anatomical part or generally relating to assessment of health of the subject. For instance, the wearable device 150 may comprise electrocardiogram (ECG) electrodes 142 being arranged on the carrier 102.

In addition, the wearable device 150 may comprise circuitry for controlling the additional sensors 140 and/or recording information acquired by the additional sensors 140. Thus, the wearable device 150 may comprise an ECG chip 144 for controlling acquisition of information by the ECG electrodes 142. The ECG chip 144 may be connected to the SoC processing unit 130 for allowing information acquired by the ECG electrodes 142 to be combined with information from the array of ultrasound transducers 110.

The wearable device 150 may also comprise an additional sensor in form of an inertial measurement unit (IMU) with associated circuitry. An IMU chip 146 may be connected to the SoC processing unit 130 for allowing information acquired by the IMU chip 146 to be combined with information from the array of ultrasound transducers 110.

The wearable device 150 may further comprise a communication unit 160 for communicating with external devices. The communication unit 160 may facilitate wired or wireless communication and may comprise an interface for wired communication, a Wireless Fidelity (Wi-Fi^{®}) communication unit and/or a Bluetooth° communication unit, such as a Bluetooth^{®} Low Energy (BLE) communication unit.

The wearable device 150 may communicate with an external device which may be arranged in any relation to the wearable device 150. For instance, the wearable device 150 may communicate with a computing device, such as a laptop, a mobile phone, or a smartwatch, which may be arranged in vicinity of the wearable device 150. The wearable device 150 may also or alternatively be configured to communicate with an external device which may be arranged anywhere, such as via communication over a telecommunication or computer network with a server "in the cloud".

It should be realized that the processing unit 130 of the system 100 may be at least partly implemented in a server or in another computing device external to the wearable device 150.

As indicated in Fig. 7a, the wearable device 150 may be configured to communicate with a server 162 "in the cloud" for providing database and analytics functionality. The wearable device 150 may further be configured to communicate with a recording system 164, such as an electronic patient record (EPR) or a health information system (HIS), for recording data relating to the health of the subject. The wearable device 150 may also be configured to communicate with a patient monitor system 166, which may be configured to detect changes to the health of the subject and provide corresponding alerts.

The wearable device 150 may also be configured to communicate with a computing device 168 which may provide an external graphical user interface (GUI) for allowing control of the system 100.

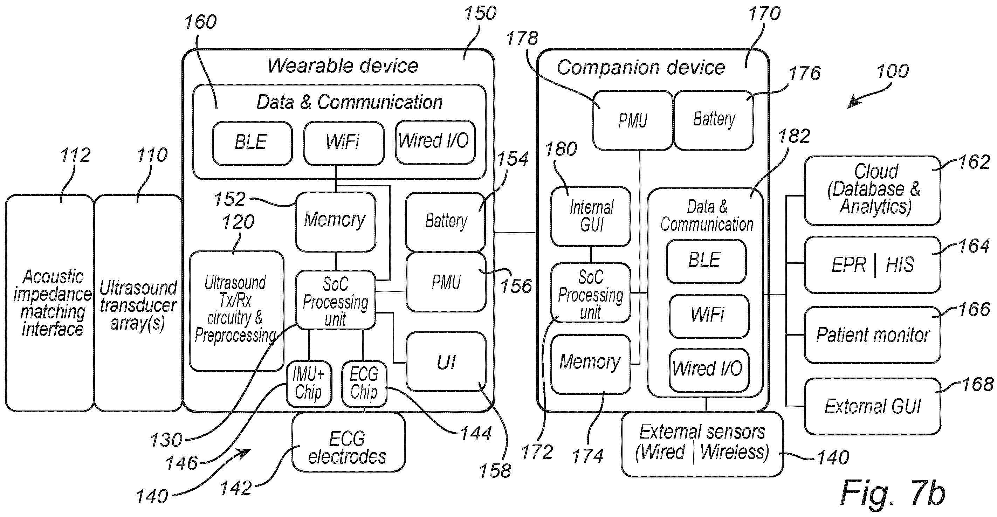

As illustrated in Fig. 7b, the system 100 may, in addition to the wearable device 150 described in relation to Fig. 7a above, also comprise a companion device 170. The companion device 170 may also be configured as a wearable to be worn by the subject. The companion device 170 may comprise additional sensor(s) 140 for acquiring further information relating to the subject. The companion device 170 may thus be configured to be arranged on a body part of the subject for placing the additional sensor(s) in a suitable location in relation to the subject.

Thanks to the companion device 170 being separate from the wearable device 150, the additional sensor(s) 140 may be arranged in a different location from the location of the wearable device 150.

The wearable device 150 may comprise all components described above in relation to Fig. 7a providing the functionality described above. However, it should be realized that some functionality may be distributed between the wearable device 150 and the companion device 170. For instance, processing of acquired information may mainly take place in the companion device 170. Also, the communication unit 160 of the wearable device 150 may be configured to solely communicate with the companion device 170, which may then be configured to communicate with external devices.

Thus, as shown in Fig. 7b, the companion device 170 may comprise an additional sensor 140. The system 100 may comprise one or more additional sensors 140 in addition to the array of ultrasound transducers 110, wherein the additional sensors 140 are associated with the wearable device 150 and/or the companion device 170.

For instance, the additional sensor(s) 140 may comprise one or more additional sensors 140 selected from the group of an electrocardiogram sensor, an inertial measurement unit, a blood pressure sensor, and a tonometer. The one or more additional sensor(s) 140 may thus be arranged on a common carrier 102 with the array of ultrasound transducers 110 and/or on a separate carrier, such as a carrier of the companion device 170.

The companion device 170 may further comprise a SoC processing unit 172, which may partially implement the functionality of the processing unit 130 discussed above and/or may provide other processing functionality.

The companion device 170 may further comprise memory 174 storing parameters and/or software to be executed by the SoC processing unit 172. The memory 174 may also be used for storing, at least temporarily, any information acquired by sensors of the system 100.

The companion device 170 may further comprise a battery 176 and a PMU 178 for controlling powering of components of the system 100.

The companion device 170 may further comprise a user interface 180, which may provide a simple interaction with a user, e.g., through buttons, LEDs, display, speakers and/or vibrotactile elements but which may also or alternatively provide a GUI.

The companion device 170 may further comprise a communication unit 182 for communicating with the wearable device 150 and/or external devices, such as the external devices described above in relation to Fig. 7a. The communication unit 182 may facilitate wired or wireless communication, e.g., in a similar manner as described above for the wearable device 150.

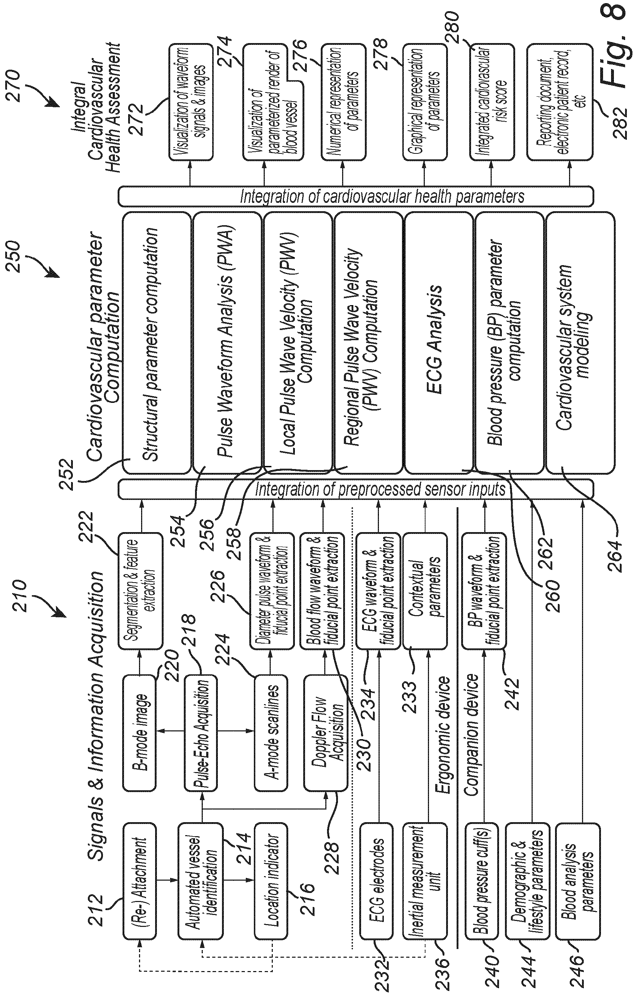

Referring now to Fig. 8, cardiovascular health assessment based on the system 100 will be described. In Fig. 8, functionalities provided by the system 100 are illustrated. These functionalities may be provided in the wearable device 150, the companion device 170, and/or external device(s). It should be realized that the system 100 may be set up to provide some or all of the functionalities illustrated in Fig. 8.

The system 100 may thus comprise a signal and information acquisition part 210, a cardiovascular parameter computation part 250, and a cardiovascular health assessment part 270.

Thus, the signal and information acquisition part 210 may comprise functionality for attachment 212 of the wearable device 150 with a possibility to adjust the position of the wearable device 150. The wearable device 150 may be configured to provide automated vessel identification and localization 214 using the array of ultrasound transducers 110. This may provide input to a location indicator 216 for determining the location of the blood vessel. The location indicator 216 may further provide feedback for adjusting the position of the wearable device 150.

Once the blood vessel is localized, the wearable device 150 may further provide pulse echo acquisition 218 for acquiring spatial information of the blood vessel. The pulse echo data may be acquired by adjacent sub-arrays for providing a B-mode image 220. The B-mode image(s) may further be processed for segmentation and feature extraction 222 relating to the blood vessel.

The pulse echo data may also or alternatively be acquired by one or more distributed sub-arrays for acquiring A-mode scanlines 224. The A-mode scanlines may be used for determining diameter pulse waveform and/or for fiducial point extraction 226.

The array of ultrasound transducers 110 may also or alternatively be used for Doppler flow acquisition 228 for providing flow information. This may be used for determining blood flow or blood flow velocity waveform and/or for fiducial point extraction 228.

The wearable device 150 may further provide ECG electrodes 142. Thus, the signal and information acquisition part 210 may comprise placement 232 of the ECG electrodes 142 by the wearable device 150. The ECG electrodes 142 may be used for acquiring ECG waveform and for fiducial point extraction 234.

ECG may provide baseline cardiovascular reference signal with fiducial points like the R-peak delimiting the cardiac cycle. It is thereby relevant for signal fusion and processing of vascular waveforms.

The ECG information may determine a phase of the cardiac cycle, which may be used for ultrasound signal acquisition to determine a point in time when locations of the vessel walls are identified.

The wearable device 150 may further provide an IMU 236. Signals from the IMU may serve as quality indicator and trigger for localization and identification of the blood vessel. The IMU may further be used for acquiring contextual parameters 238 that are of physiological relevance, providing input of activity of the subject or orientation of the subject, such as whether the subject is standing or supine during measurements by other sensors.

The signal and information acquisition part 210 may further use additional sensors 140, which may for instance be arranged in the companion device 170. Thus, the signal and information acquisition part 210 may use blood pressure cuff(s) 240 in the companion device 170. The blood pressure cuff(s) may be used for determining blood pressure waveform and/or for fiducial point extraction 242.

The companion device 170 may also be used for acquisition via a user interface of demographic and lifestyle parameters 244 and/or blood analysis parameters 246.

The sensor input and/or preprocessed sensor input providing information acquired in the signal and information acquisition part 210 may be integrated and provided to the cardiovascular parameter computation part 250. The cardiovascular parameter computation part 250 may comprise one or more units as described below, which may each use one or more of the inputs provided from the signal and information acquisition part 210.

Thus, the cardiovascular parameter computation part 250 may be configured to perform structural parameter computation 252. This may for instance be based on B-mode images for computation and determination of structural vessel parameters like diameter, stenosis, intima-media thickness, and vascular dissections.

The cardiovascular parameter computation part 250 may further be configured to perform pulse waveform analysis (PWA) 254. The PWA may be performed on diameter and pressure waveforms to determine an augmentation index, or stiffness/reflection index, etc.

The PWA may further perform waveform intensity analysis, combining flow and diameter/pressure waveforms to assess intensity, reflections, reservoir, etc. Thanks to the signal and information acquisition part 210 providing spatially distributed ultrasound transducers 110, local wave directions may be assessed.

The cardiovascular parameter computation part 250 may further be configured to perform local pulse wave velocity (PWV) computation 256. This may be performed based at least on the diameter pulse waveforms. The local PWV computations may be performed by modeling spatiotemporal wave propagation model (e.g., using Gaussian waveform modeling to account for wave reflections), a distensibility method (dD/dP) from diameter and pressure changes, and/or loop-based methods (e.g., Pressure / Flow, Flow / Diameter).

The cardiovascular parameter computation part 250 may further be configured to perform regional PWV computation 258, which may provide information of arterial stiffness. This may be performed via pulse arrival time segmentation, using ultrasound and ECG information, effectively yielding heart to carotid PWV, and/or by various combinations of ultrasound device(s), blood pressure cuff(s) and/or tonometer(s) providing carotid-femoral PWV, brachial-ankle PWV, and/or cardio-ankle vascular index (CAVI).

The cardiovascular parameter computation part 250 may further use blood flow waveforms for correction of blood flow impact on PWV and/or for computation of volume flow and cardiac output (in combination with diameter waveforms).

The cardiovascular parameter computation part 250 may further be configured to perform ECG analysis 260. This may be based on the ECG information for providing R-peak to R-peak interval computation, atrial fibrillation detection and/or arrhythmia computation.

The cardiovascular parameter computation part 250 may further be configured to perform blood pressure parameter computation 262. This may be based on information acquired by the ultrasound transducers 110 and/or the blood pressure cuff(s).

The blood pressure parameter computation 262 may be configured to determine an absolute blood pressure and/or to monitor blood pressure changes.

The blood pressure parameter computation 262 may be configured to provide multi-site blood pressure measurement using cuffs, central blood pressure via calibration of central diameter waveforms to brachial blood pressure and using modeling, central blood pressure computation via brachial cuff blood pressure and central ultrasound-based diameter waveform information, e.g., PWV.

The blood pressure parameter computation 262 may be configured to provide cuff-less blood pressure computation (absolute and relative) via integral model based on all or some functional and structural parameters available, such as pulse waveform analysis of diameter and flow waveforms, local and regional PWV, and arterial diameter and wall thickness.

The cardiovascular parameter computation part 250 may further be configured to perform cardiovascular system modeling 264 in zero to three dimensions. The cardiovascular system modeling 264 may use flow and diameter/pressure waveforms for the modeling.

The cardiovascular system modeling 264 may be configured to provide compliance / resistance assessment, diastolic resistor-capacitor (RC) time constant assessment, and/or blood flow modeling.

The cardiovascular health parameters determined in the cardiovascular parameter computation part 250 may be integrated and provided to the cardiovascular health assessment part 270. The cardiovascular health assessment part 270 may comprise one or more units as described below, which may each use one or more of the parameters provided from the cardiovascular parameter computation part 210.

The cardiovascular health assessment part 270 may be configured to provide presentation of the cardiovascular health parameters so as to facilitate assessment of cardiovascular health.

Thus, the cardiovascular health assessment part 270 may be configured to provide visualization of waveform signals and images 272, visualization of parameterized render of blood vessel 274, numerical representation of parameters 276, possibly in relation to normative and/or threshold values, graphical representation of parameters 278, possibly in relation to normative and/or threshold values, an integrated cardiovascular risk score 280, possibly in relation to normative and/or threshold values, and/or output of a reporting document, electronic patient record, or the like 282.

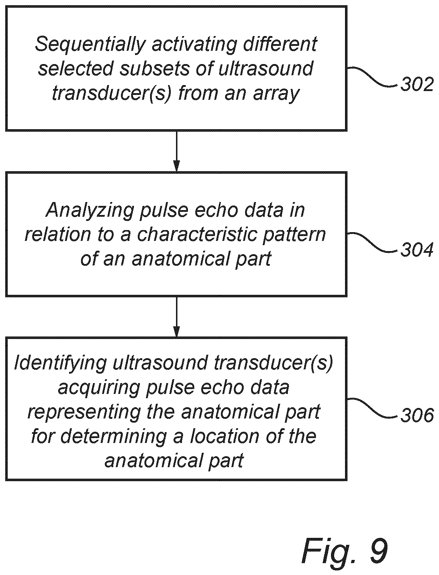

Referring now to Fig. 9, a method for localization of an anatomical part in a subject will be summarized.

The method comprises sequentially activating 302 different selected subsets of at least one ultrasound transducer from an array of ultrasound transducers for controlling the subset to assume a localization mode. Each ultrasound transducer in the selected subset is configured to transmit one or more pulses of ultrasound signals into the subject and to detect the reflected one or more pulses for forming pulse echo data representing an ultrasound response from the subject for each pulse as a function of a time delay of the ultrasound response.

The method further comprises analyzing 304 the pulse echo data acquired by a plurality of selected subsets in relation to a characteristic pattern of pulse echo representing a structure of the anatomical part.

The method further comprises identifying 306 one or more ultrasound transducers being located to acquire pulse echo data representing the anatomical part for determining a location of the anatomical part in the subject.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A system (100) for localization of an anatomical part in a subject, said system (100) comprising:
an array of ultrasound transducers (110) configured to transmit ultrasound signals into the subject and to detect reflected ultrasound signals from the subject;
a control unit (120) configured to activate a selected subset of at least one ultrasound transducer (110) from the array of ultrasound transducers for controlling the subset to assume a localization mode, wherein the at least one ultrasound transducer (110) in the selected subset is configured to transmit one or more pulses of ultrasound signals into the subject and to detect the reflected one or more pulses for forming pulse echo data representing an ultrasound response from the subject for each pulse as a function of a time delay of the ultrasound response, wherein the control unit (120) is further configured to sequentially activate different selected subsets from the array of ultrasound transducers (110);
a processing unit (130) configured to analyze the pulse echo data acquired by a plurality of selected subsets in relation to a characteristic pattern of pulse echo representing a structure of the anatomical part, and to identify one or more ultrasound transducers (110) being located to acquire pulse echo data representing the anatomical part for determining a location of the anatomical part in the subject.

2. The system according to claim 1, further comprising:
a carrier (102) configured to be adjustably positioned in relation to a body part, such as around the body part, wherein the array of ultrasound transducers (110) is configured to be carried by the carrier (102) for placing the array of ultrasound transducers (110) in relation to the anatomical part.

3. The system according to claim 2, wherein the carrier (102) is configured to be strapped around the body part.

4. The system according to claim 2 or 3, wherein the processing unit (130) is configured to determine a direction of adjustment of a position of the carrier (102) for improving placement of the array of ultrasound transducers (110) in relation to the anatomical part.

5. The system according to any one of the preceding claims, wherein the processing unit (130) is configured to analyze the pulse echo data in relation to the characteristic pattern of pulse echo representing the structure of a blood vessel.

6. The system according to claim 5, wherein the processing unit (130) is configured to analyze the pulse echo data in relation to the characteristic pattern including two amplitude peaks separated by a hypoechoic portion.

7. The system according to any one of the preceding claims, wherein the control unit (120) is configured to control the selected subset to transmit and/or receive the ultrasound signals in plane wave mode or in focused mode.

8. The system according to any one of the preceding claims, wherein the subset of at least one ultrasound transducer (110) is configured to, in the localization mode, transmit a plurality of pulses of ultrasound signals and to detect a plurality of reflected pulses, wherein the processing unit (130) is configured to analyze the pulse echo data to identify a pulsatile change of the structure of the anatomical part for identifying that pulse echo data represents the anatomical part.

9. The system according to any one of the preceding claims, wherein the control unit (120) is further configured to, based on the location of the anatomical part, activate one or more subsets of ultrasound transducers (110) for controlling the one or more subsets to assume a spatial information acquisition mode, wherein each ultrasound transducer (110) in the one or more subsets is configured to transmit one or more pulses of ultrasound signals into the subject and to detect the reflected one or more pulses for forming pulse echo data representing spatial information of the anatomical part.

10. The system according to any one of the preceding claims, wherein the control unit (120) is further configured to, based on the location of the anatomical part, activate one or more subsets of ultrasound transducers (110) for controlling the one or more subsets to assume a flow information acquisition mode, wherein each ultrasound transducer (110) in the one or more subsets is configured to transmit ultrasound signals into the subject and to detect the reflected ultrasound signals for representing Doppler frequency and/or Doppler shift based on a flow in the anatomical part.

11. The system according to any one of the preceding claims, wherein the system (100) is further configured to acquire arterial information of an artery in the anatomical part, and wherein the processing unit (130) is configured to process the arterial information for determining at least one parameter for characterization of the artery, such as the at least one parameter being pulse wave velocity.

12. The system according to any one of the preceding claims, further comprising one or more additional sensors (140) selected from the group of an electrocardiogram sensor, an inertial measurement unit, a blood pressure sensor, and a tonometer.

13. The system according to any one of the preceding claims, wherein the system (100) is configured to detect at least one parameter for estimating blood pressure based on at least one parameter.

14. The system according to any one of the preceding claims, wherein the control unit (120) is further configured to control the array of ultrasound transducers (110) to transmit a targeted pulse based on the localization of the anatomical part in the subject, such as controlling the array of ultrasound transducers (110) to transmit the targeted pulse for stimulating the anatomical part and/or controlling the array of ultrasound transducers (110) to transmit the targeted pulse for mechanically characterizing tissue of the anatomical part.

15. A method for localization of an anatomical part in a subject, said method comprising:
sequentially activating (302) different selected subsets of at least one ultrasound transducer from an array of ultrasound transducers for controlling the subset to assume a localization mode, wherein each ultrasound transducer in the selected subset is configured to transmit one or more pulses of ultrasound signals into the subject and to detect the reflected one or more pulses for forming pulse echo data representing an ultrasound response from the subject for each pulse as a function of a time delay of the ultrasound response;
analyzing (304) the pulse echo data acquired by a plurality of selected subsets in relation to a characteristic pattern of pulse echo representing a structure of the anatomical part, and
identifying (306) one or more ultrasound transducers being located to acquire pulse echo data representing the anatomical part for determining a location of the anatomical part in the subject.
